# EUROPEAN PATENT SPECIFICATION

(11) **EP 3 373 973 B1**
(45) Date of publication and mention of the grant of the patent: **29.06.2022**
(21) Application number: 16794332.3
(22) Date of filing: 10.11.2016
(51) Int. Cl.: A61K 9/06, A61K 9/16, A61K 9/51, A61K 47/02, A61K 47/08, A61K 47/10, A61K 47/18, A61K 47/20, A61K 47/22, A61K 47/26, A61K 47/34, A61K 47/42, A61K 31/573, A61K 31/58, A61K 38/38, A61K 39/395

(54) **OCULAR COMPOSITIONS**
ZUSAMMENSETZUNGEN FÜR DAS AUGE
COMPOSITIONS OCULAIRES

(30) Priority: 10.11.2015 GB 201519811
(43) Date of publication of application: 19.09.2018
(73) Proprietor: Re-Vana Therapeutics Ltd., Belfast BT7 1NF (GB)
(72) Inventor: THAKUR, Raghu Raj Singh, Belfast Antrim BT7 1NF (GB); JONES, David, Belfast Antrim BT7 1NF (GB); GUJRAL, Chirag, Belfast Antrim BT7 1NF (GB)
(74) Representative: FRKelly
(86) International application number: PCT/EP2016/077269
(87) International publication number: WO 2017/081154

(56) References cited:
- EP-A1- 2 481 399
- WO-A1-2005/110362
- WO-A1-2010/005290
- WO-A2-2009/073193
- LU CHANGHAI ET AL: "Hydrogel containing silica shell cross-linked micelles for ocular drug delivery", JOURNAL OF PHARMACEUTICAL SCIENCES, vol. 102, no. 2, February 2013 (2013-02), pages 627-637, XP002766597,

## Description

### BACKGROUND OF THE INVENTION

Chronic retinal diseases are the leading contributor to visual impairment and blindness worldwide. Loss of sight has a major personal impact on people's daily lives and has a profound economic impact on individuals, families, support agencies, society and the state. The World Health Organization estimates that globally about 285 million people are visually impaired, of which 39 million are blind and 246 million have low vision. Diseases that originate in the posterior segment (PS) or back of the eye lead to permanent loss of vision if left untreated and account for the majority of blindness, such as in age-related macular degeneration (AMD), diabetic retinopathy (DR), diabetic macular edema (DME), cytomegalovirus (CMV) retinitis, retinitis pigmentosa, uveitis and glaucoma. The PS of the eye, which includes the retina, choroid, and vitreous body, is difficult to access due to the recessed location within the orbital cavity. Therefore, drug delivery to the PS of the eye has remained one of the most challenging tasks for pharmaceutical scientists and retina specialists. Multiple approaches have been used to deliver drugs to the PS of the eye such as systemic, topical, periocular (or transscleral) and intravitreal. Topical (e.g. eye drops) and systemic (e.g. oral tablets) routes result in low or sub-therapeutic drug levels due to multiple ocular barriers, requiring administration of unnecessarily high concentrations of drug that causes drug-related toxicity and producing low treatment efficacy. The periocular route involves injections on the outer surface of the eye. Depending upon their area of injection they are termed as subconjunctival, peribulbar, subtenon and retrobulbar injections. Following injection, transient diffusion of drug occurs across the large white structural sheath around the circumference of the eye. Drug diffusion across the scleral membrane is dependent upon drug's solubility, molecular weight/molecular radius, charge and polarity. However, this method has shown low intraocular bioavailability due to a delay in drug diffusion through the sclera, systemic clearance and loss of drug before reaching the target tissues (e.g. retina/choroid). One of the standard treatments to prevent the above chronic conditions is by frequent intravitreal injections (direct injection into the eye) of drug solutions (e.g. ranibizumab, bevacizumab, triamcinolone acetonide and dexamethasone) using traditional hypodermic needles. Intravitreal injections have the advantage of delivering drugs directly to the required site, unlike conventional topical or systemic routes. However, this is not a desirable method of drug delivery for several reasons: the need for frequent injections, significant tissue trauma, short half-lives of injected drugs, uncomfortable and painful to patients, requires professional training, causes rise in intraocular pressure (IOP), can result in severe injection-related infections (e.g. endophthalmitis, hemorrhage, and cataract), mechanical injury to the lens and retina, high drug-induced toxicities, and higher costs. Furthermore, post intravitreal injection patients may require hypotensive treatment to maintain normal IOP, and may even undergo glaucoma surgery. These risks are dependent upon the needle type, where lower gauge needles causes higher damage to the eye. Therefore, reducing the dosing frequency is the greatest, realistic unmet need in treating these diseases. Hence, there is a great need to develop long-acting controlled release delivery systems. To this end, limited controlled release intraocular implants have been engineered that are either injected with hypodermic needles, or surgically sutured to the eye, yet these methods of administration are highly invasive and cause excessive tissue trauma. For example: Vitrasert^{®} (ganciclovir implant), Retisert^{®} (fluocinolone acetonide implant), lluvien^{®} (fluocinolone acetonide implant), and Ozurdex^{™} (dexamethasone implant) that are commercially available to treat cytomegalovirus retinitis, uveitis, DME and macular edema/uveitis, respectively. Other products under development include; I-Vation^{™} for AMD and DME; Rh CNTF for retinal pigmentation and AMD; Visulex^{®} for uveitis; and Verisome^{™} for AMD and DR indications. Vitrasert^{®}, Retisert^{®}, I-Vation^{™} and Rh CNTF are non-biodegradable and surgically implanted in the eye, with attendant higher risks for infections, higher cost of administration, increased IOP and low patience compliance. Also, these implants require a secondary surgical procedure to either remove or replace with a new implant. EP 2 481 399 A1, for instance, discloses a slow-releasing ocular implant prepared by mixing collagen impregnated with a drug, a photocurable polyethylene glycol and a photoinitiator, and curing the mixture with UV light. Iluvien^{®} (non-biodegradable) & Ozurdex^{®} (biodegradable) are injected into the eye by using 25G and 22G needles, respectively, a procedure that takes ~20 minutes to accomplish, causing considerable pain/discomfort and significant morbidity (subconjunctival haemorrhage, vitreous leak and increased IOP). Alternatively, non-invasive devices for sustained transscleral delivery such as iontophoresis, electroporation, electrophoresis, and photoacoustic could avoid surgical intervention to significantly improve patient compliance. However, wear time of these devices (e.g. 5 to 20 min for iontophoresis), degree of discomfort to patients, potential for adequate sustained release, costs and safety of long-term application are yet to be established. Furthermore, no long-acting or controlled release protein/peptide-based therapies have gained approval in an implant system to date.

Therefore, there is a need for new and improved systems for ocular delivery of therapeutic agents.

### SUMMARY OF THE

The invention is defined by the claims. Any subject-matter falling outside the scope of the claims is provided for information purposes only.

The present invention provides ocular compositions that can be administered to the eye in various forms to achieve controlled release of a therapeutic agent (or drug). The invention allows the flexibility to administer a range of small and large drug molecules including proteins, peptides and gene therapeutics, and maintain their activity for a controlled period of time.

According to a first aspect of the invention, there is provided an injectable ocular composition comprising:
i) 99 to 60 % (w/w) of a photopolymerizable composition selected from the group consisting of polyalkylene glycol diacrylate and polyalkylene glycol dimethacrylate, wherein the photopolymerizable composition has a molecular weight in the range of 200 to 20,000 Dalton;
ii) a biodegradable polymer selected from the group consisting of lactide/glycolide copolymer (PLGA), polylactic acid (PLA), polyglycolic acid (PGA), polycaprolactone (PCL), lactide/caprolactone copolymer (PLC), poly (L-lactide) (PLLA) and mixtures, copolymers, and block copolymers thereof;
iii) a photoinitiator; and
iv) a therapeutic agent.

The ocular composition may be for use to form an ocular implant or for use to coat an ocular implant, optionally wherein the implant is an *in situ* formed ocular implant.

The implant may be an *in situ* formed ocular implant, wherein the photopolymerizable composition has a molecular weight in the range of 200 to 20,000 Dalton, more preferably in the range of 200 to 3,000 Dalton or 200 to 1,000 Dalton.

The implant may be a pre-formed ocular implant.

In the ocular composition described above, the biodegradable polymer may be PLGA or the biodegradable polymer may be selected from the group PCL, PLC, PLLA, and mixtures, copolymers, and block copolymers thereof.

In the ocular composition described above, the photopolymerizable composition may be polyalkylene glyco diacrylate or may be selected from the group consisting of polyethylene glycol diacrylate, diethylene glycol diacrylate, polyethylene glycol dimethacrylate, diethylene glycol dimethacrylate, polypropylene glycol diacrylate, dipropylene glycol diacrylate, dipropylene glycol dimethacrylate, and polypropylene glycol dimethacrylate; or may be polyethylene glycol diacrylate or polyethylene glycol dimethacrylate; or may be polyethylene glycol diacrylate.

The molar ratio of lactic acid to glycolic acid in the PLGA may be 90% lactic acid to 10% glycolic acid, 85% lactic acid to 15% glycolic acid, 75% lactic acid to 25% glycolic acid, 65% lactic acid to 35% glycolic acid, 50% lactic acid to 50% glycolic acid, 35% lactic acid to 65% glycolic acid, 25% lactic acid to 75% glycolic acid, 15% lactic acid to 85% glycolic acid, and 10% lactic acid to 90% glycolic acid.

In one embodiment, the ocular composition comprises
i) 79.5 to 59.5 % (w/w) polyethylene glycol diacrylate or polyethylenene glycol dimethacrylate; and
ii) 20 to 40 % (w/w) PLGA, wherein the molar ratio of lactic acid to glycolic acid in the PLGA is 90% lactic acid to 10% glycolic acid, 85% lactic acid to 15% glycolic acid, 75% lactic acid to 25% glycolic acid, or 50% lactic acid to 50% glycolic acid; or

i) 69.5 % (w/w) polyethylene glycol diacrylate or polyethylene glycol dimethacrylate; and
ii) 30 % (w/w) PLGA wherein the molar ratio of lactic acid to glycolic acid in the PLGA is 90% lactic acid to 10% glycolic acid, 85% lactic acid to 15% glycolic acid, 75% lactic acid to 25% glycolic acid, or 50% lactic acid to 50% glycolic acid; or

i) 95.5 to 84.5 % (w/w) polyalkylene glycol diacrylate or polyalkylene glycol dimethacrylate; and
ii) 4 to 15 % (w/w) PCL; or

i) 79.5 to 94.5 % (w/w) polyalkylene glycol diacrylate or polyalkylene glycol dimethacrylate; and
ii) 5 to 20 % (w/w) PLLA.; or

i) 95.5 to 84.5 % (w/w) polyalkylene glycol diacrylate or polyalkylene glycol dimethacrylate; and
ii) 4 to 15 % (w/w) PLC in which lactic acid to caprolactone is in the range of 90 % lactic acid to 10% caprolactone, 80 % lactic acid to 20 % caprolactone, 70 % lactic acid to 30 % caprolactone, 60 % lactic acid to 40 % caprolactone, or 50 % lactic acid to 50 % caprolactone.

The ocular composition may further comprise a solvent selected from dimethyl sulfoxide, decylmethyl sulfoxide, 2-pyrrolidone, 1-methyl-2-pyrrolidne, N-vinyl-pyrrolidine, N-Methyl-2-pyrrolidone, N-ethyl-pyrrolidone, glycerol formal, glycerol, polyethylene glycol, propylene glycol, benzyl alcohol, benzyl benzoate, ethyl benzoate, triacetin, triethyl citrate, dimethylformamide, dimethylacetamide and tetrahydrofuran. Preferably the solvent is selected from dimethyl sulfoxide, decylmethyl sulfoxide, 2-pyrrolidone, 1-methyl-2-pyrrolidne, N-Methyl-2-pyrrolidone, and glycerol formal.

The ocular composition may further comprise a pore-forming agent; preferably wherein the pore-forming agent is selected from polyethylene glycol, maltose, glucose, agarose, mannitol, gelatin, sodium chloride, magnesium carbonate, magnesium hydroxide, potassium chloride, sodium bicarbonate, potassium bicarbonate, and sucrose.

In one embodiment, the biodegradable polymer is essentially contained within a matrix of the photopolymerizable composition.

The photoinitiator may be a hydroxyketone photoinitiator, an amino ketone photoinitiator, a hydroxy ketone/benzophenone photoinitiator, a benzyldimethyl ketal photoinitiator, a phenylglyoxylate photoinitiator, an acyl phosphine oxide photoinitiator, an acyl phosphine oxide/alpha hydroxy ketone photoinitiator, a benzophenone photoinitiator, a ribityl isoalloxazine photoinitiator, or a phenylglyoxylate photoinitiator or any combination thereof. More preferably the photoinitiator is 1-[4-(2-hydroxyethoxy)-phenyl]-2-hydroxy-2-methyl-1- propanone, 2,2-dimethoxy-2-phenylacetophenone (DMPA) or riboflavin.

The ocular composition may further comprise a co-initiator; preferably wherein the photoinitiator is riboflavin and the co-initiator is L-arginine.

The ocular composition may be a nanoparticle or a microparticle ocular implant; preferably wherein the nanoparticle ocular implant is less than 1,000 nm; preferably wherein the microparticle ocular implant is less than 1,000 µm.

The invention also provides methods of making the ocular composition as described herein. One method comprises the steps of:
i) mixing the therapeutic agent, the photopolymerizable composition, the biodegradable polymer and the photoinitiator, in any order of addition, to form mixture i);
ii) irradiating the mixture i) with light at a wavelength of between 230 to 550 nm, between 300 to 525 nm, or between 350 to 490 nm for between 1 second and 60 minutes to form the ocular composition; and
iii) wherein the composition ii) is for administration to an ocular area of a subject.

Another method comprises the steps of:
i) mixing the therapeutic agent, the photopolymerizable composition, the biodegradable polymer and the photoinitiator, in any order of addition, to form mixture i);
ii) adding the mixture i) to an aqueous medium to form mixture ii);
iii) sonicating the mixture ii); and
iv) irradiating the mixture ii) with light at a wavelength of between 230 to 550 nm, between 300 to 525 nm, or between 350 to 490 nm for between 1 second and 60 minutes to form nanoparticles or microparticles.

In the ocular composition as described herein, the photopolymerizable composition can have a molecular weight in the range of 200 to 1,000 Dalton. The invention also provides an ocular composition as described herein, obtainable by a method comprising the step of mixing the therapeutic agent, the photopolymerizable composition, the biodegradable polymer and the photoinitiator, in any order of addition, to form a mixture for use in the ocular area of a subject, wherein, after administration, the mixture is irradiated with light at a wavelength of between 230 to 550 nm, between 300 to 525 nm, or between 350 to 490 nm for between 1 second and 60 minutes to form the ocular composition.

### BRIEF DESCRIPTION OF THE DRAWINGS

In the following figures, only the injectable ocular compositions as claimed are according to the invention.

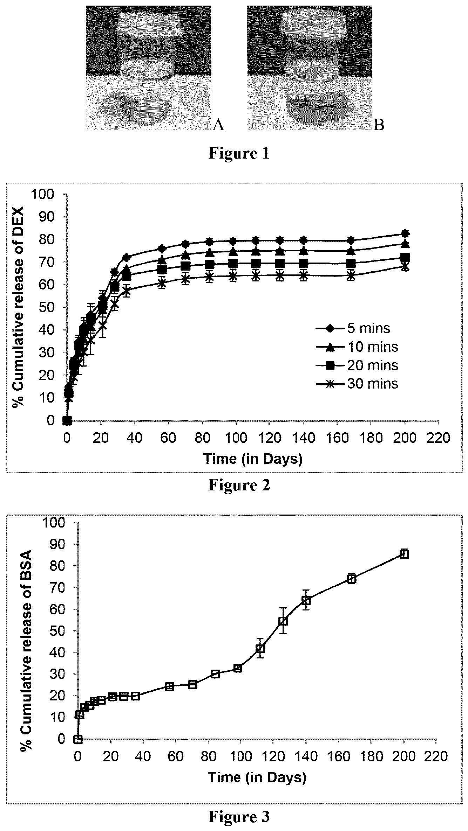

**Figure** 1: Digital images of blank (without drug) ISPcl showing (A) *in situ* implant formation in PBS and (B) degradation of implant after 160 days. The ISPcl were composed of 30%w/w PLGA 75/25, 0.1% w/w Irgacure^{®} 2959 and 69.9% w/w of PEGDA 700. The gels were *in situ* crosslinked in PBS using UV light (at 365 nm, 3.14 mW/cm²) for 5 min. Mean ± S.D, n=3.
**Figure 2****:** *In vitro* release of dexamethasone (DEX) from ISPcl containing 30%w/w PLGA 75/25, 0.5%w/w DEX, and 0.1%w/w of Irgacure^{®} 2959 and 69.4% w/w of PEGDA 700. The gels were *in situ* crosslinked, using UV light at 365 nm, for 5, 10, 20 and 30 min, respectively. Mean ± S.D, n=3
**Figure 3****:** *In vitro* release of bovine serum albumin (BSA) from ISPcl containing 30%w/w PLGA 75/25, 0.5%w/w BSA, and 0.1%w/w of Irgacure^{®}2959 and 69.4% w/w of PEGDA 700. The gels were *in situ* crosslinked, using UV light at 365 nm for 5 min. Mean ± S.D, n=3

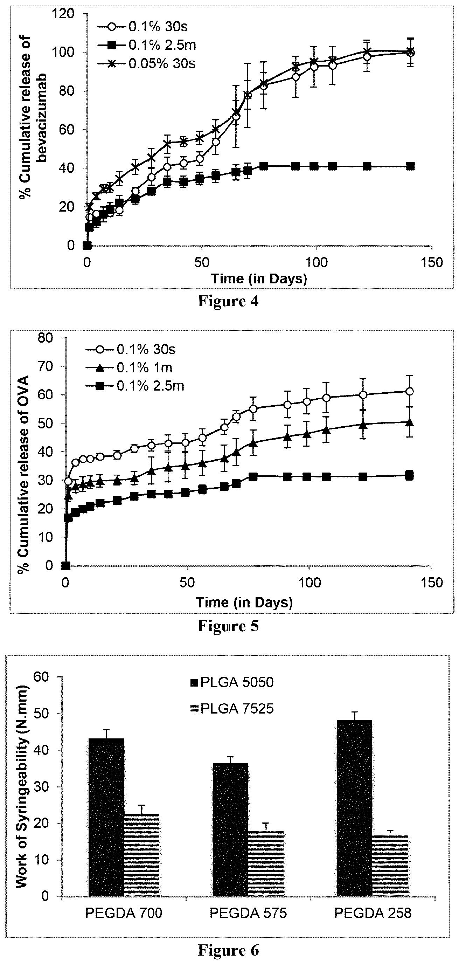

**Figure 4****:** *In vitro* release of bevacizumab (Avastin) from ISPcl containing 30% w/w PLGA 75/25, 1.5% w/w bevacizumab, 0.1% w/w Irgacure^{®} and 68.4% w/w PEGDA 700 and 30% w/w PLGA 75/25, 1.5% w/w bevacizumab, 0.05% w/w Irgacure^{®} and 68.45% w/w PEGDA 700. *In situ* crosslinked using UV light at 365 nm for either 30 sec or 2.5 min. Mean ± S.D, n=3
**Figure 5****:** *In vitro* release of ovalbumin (OVA) from ISPcl containing 30%w/w PLGA 75/25, 2.5% w/w OVA and 0.1% w/w Irgacure^{®} 2959 and 67.4%w/w of PEGDA 700. *In situ* crosslinked for either 30 sec, 1 min or 2.5 min. Mean ± S.D, n=3.
**Figure 6****:** Work of Syringeability (WoS) of 30%w/w PLGA50/50 and 30%w/w PLGA75/25 and 0.1% w/w Irgacure^{®} 2959 in formulations containing different molecular weights of 69.9% w/w of PEGDAs (258, 575 and 700). The WoS was calculated from the resultant of force-distance plots Mean ± S.D, n=5.

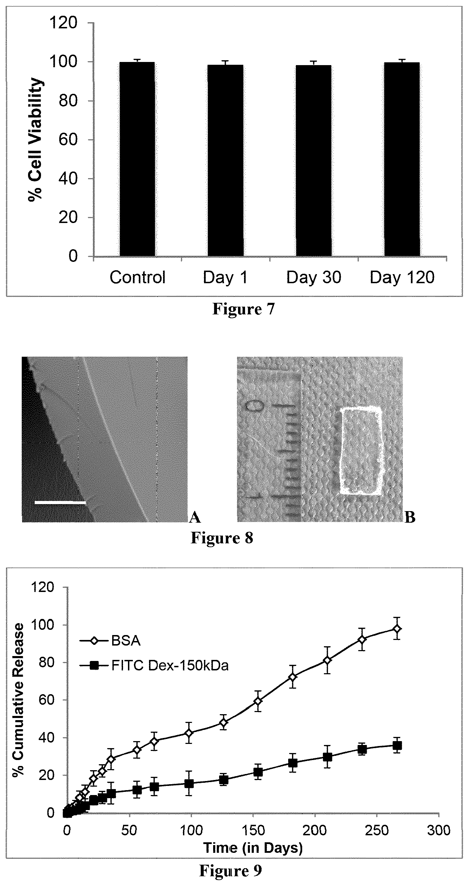

**Figure 7****:** Percentage cell viability of human retinal pigment epithelial cell line (ARPE-19) following exposure of the release media, at different intervals, of the ISPcl that were composed of 30%w/w PLGA 75/25 0.1%w/w of Irgacure^{®} 2959 and 69.9% w/w PEGDA 700 and UV crosslinked for 5 min. Mean ± SD, n=3.
**Figure 8:** (A) Scanning electron microscope image of PPcl implant (scale bar is 50 µm). (B) Digital image of a PPcl adjacent to a ruler.
**Figure 9****:** *In vitro* release of FITC-Dextran 150kDa and BSA from PPcl containing 30%w/w of PLGA 75/25, 0.5%w/w FITC-Dextran 150kDa or BSA and 0.1%w/w of Irgacure^{®} 2959 and 69.4% w/w PEGDA 700. These PPcl were cross-linked using UV curing system at wavelength 365 nm for 5 runs at a lamp intensity of 100%.

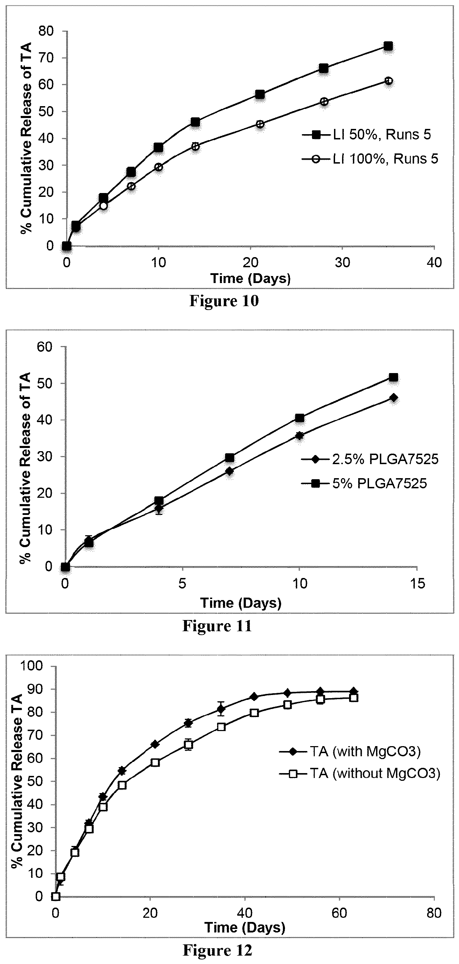

**Figure 10****:** *In vitro* release of TA from PPcl containing 30%w/w of PLGA 75/25, 2.5%w/w TA, and 0.1%w/w of Irgacure^{®} 2959 and 67.4%w/w PEGDA 700. These PPcl were cross-linked using UV curing system at wavelength 365nm for 5 runs, at a lamp intensity (LI) of 50% and 100%.
**Figure 11****:** *In vitro* release of TA from PPcl containing 2.5 or 5 %w/w of PLGA 75/25, 2.5%w/w TA, 0.1%w/w of Irqacure^{®} 2959 in 94.9% w/w or 92.4% w/w of PEGDA 700, respectively. These PPcl were cross-linked using UV curing system at wavelength 365nm for 5 runs, at a lamp intensity (LI) of 25%.
**Figure 12****:** *In vitro* release of TA from PPcl containing 2.5 %w/w of PLGA 75/25, 2.5%w/w TA, 0.1%w/w of Irgacure^{®}2959 and without pore forming agent in 94.9%w/w PEGDA 700 or with 2%w/w of pore forming agent (i.e. MgCO₃) in 92.9%w/w PEGDA 700. These PPcl were cross-linked using UV curing system at wavelength 365 nm for 10 runs, at a lamp intensity (LI) of 25%.

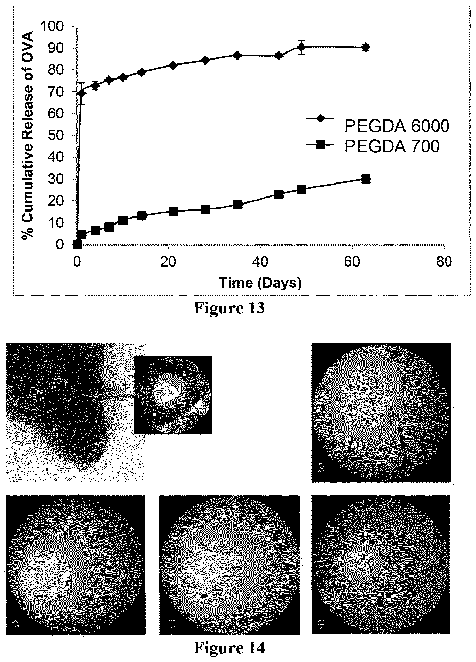

**Figure 13****:** *In vitro* release of TA from PPcl containing 2.5 %w/w of PLGA 75/25, 2.5%w/w TA, 0.1%w/w of Irgacure^{®} 2959 in 94.9%w/w of PEGDA 700 or PEGDA 6000. These PPcl were cross-linked using UV curing system at wavelength 365 nm for 10 runs, at a lamp intensity (LI) of 25%.
**Figure 14****:** *In vivo* implant formation of ISPcl in rat eye following intravitreal injection (A) Digital image of rat following administration of fluorescein sodium loaded ISPcl implant (2 µL), the inset shows close-up image of the implant in the eye, (B) shows fundus image of control eye without any implant, (C-E) fundus images showing the time course of fluorescein sodium release from ISPcl implant, taken on (C) day 1, (D) day 6 and (E) day 18. Implants indicate slow and continuous release of fluorescein sodium and degradation over the time.

### DETAILED DESCRIPTION

### Photopolymerizable Compositions

The photopolymerizable polymers of the present invention can be used in any of the compositions and implants of the invention in combination with any of the claimed biodegradable polymers, and with any therapeutic agents, photoinitiators, solvents, co-solvents, pore forming agents, and co-initiators described herein or known in the common general knowledge.

In one embodiment, the photopolymerizable compositions of the invention can be biodegradable. As used herein, "biodegradable" is the chemical degradation by biological means. In some embodiments, the biodegradation is about 100%, about 98%, about 90%, about 85%, about 80%, about 60%, about 50%, or about 45% degradation of one or more of the compositions, monomers, oligomers, fragments, polymers, photoinitiators, solvents, co-solvents, or co-initiators. In some embodiments the biodegradation takes place over about 1 minute, about 10 minutes, about 20 minutes, about 2 hours, about 6 hours, about 12 hours, about 24 hours, about 2 days, about 5 days, about 1 week, about 1 month, about 2 months, about 5 months, about 6 months, about 8 months or about 12 months. In some embodiments the biodegradation takes place between about 1 month and about 12 months, between about 6 months and about 12 months, or between about 8 months and about 12 months.

As used herein, the term "photopolymerizable composition" is a composition which can form a crosslinked polymer network upon exposure to light, in particular UV light. As used herein, photopolymerizable compositions include photopolymerizable monomers and oligomers (such as, dimers, trimers, and tetramers). The terms "oligomers" and "fragments" can be used interchangeably to mean between two and twenty monomers, optionally between two and ten monomers, further optionally between two and five monomers or between two and four monomers. A "photopolymerizable monomer" is a single unit of a photopolymerizable polymer that can bind chemically to other monomers to form a polymer.

Photopolymerizable compositions of the present invention can be crosslinked with UV radiation to form the photopolymerized polymers of the present invention.

The photopolymerizable compositions of the present invention are selected from the group consisting of polyalkylene glycol diacrylate and polyalkylene glycol dimethacrylate as claimed. In one embodiment, the photopolymerizable composition is polyalkylene glycol diacrylate or is selected from the group consisting of polyethylene glycol diacrylate, diethylene glycol diacrylate, polyethylene glycol dimethacrylate, diethylene glycol dimethacrylate, polypropylene glycol diacrylate, dipropylene glycol diacrylate, dipropylene glycol dimethacrylate, and polypropylene glycol dimethacrylate; or is polyethylene glycol diacrylate or polyethylene glycol dimethacrylate; or is polyethylene glycol diacrylate

The molecular weight of the photopolymerizable compositions of the present invention is in the range of 120,000 Da, in the range of 200 to about 10,000 Da, between 200 and about 8,000 Da, between 200 and about 5,000 Da, or between 200 and about 1,000 Da.

It has been found, for the compositions and implants of the present invention, that an increase in molecular weight of the photopolymerizable compositions results in an increase in drug release rate. Without wishing to be bound by theory, it is believed that photopolymerizable compositions with lower molecular weights have higher crosslinking density and therefore slower drug release rates.

The photopolymerizable compositions of the present invention typically have viscosities between about 0.1 to about 7 dL/g, between about 0.2 to about 5 dL/g, or between about 0.5 to 2 dL/g.

In another embodiment, the photopolymerizable compositions of the present invention are polymerized by irradiating the composition with light at a wavelength of between about 230 to about 550 nm, between about 300 to about 525 nm, or between about 350 to about 490 nm for between about 1 second and about 60 minutes, between about 30 seconds and about 30 minutes, between about 2.5 minutes and about 20 minutes, for between about 5 minutes and about 10 minutes. In one embodiment, the crosslinking is for about 30 seconds, about 1, about 2.5, about 5, about 10, about 20 or about 30 minutes.

### Biodegradable Polymers

The biodegradable polymers of the present invention can be used in any of the compositions and implants of the invention in combination with any of the claimed photopolymerizable compositions, and with any therapeutic agents, photoinitiators, solvents, co-solvents, pore forming agents, and co-initiators described herein or known in the common general knowledge.

The biodegradable polymers of the present invention are biodegradable but not photopolymerizable.

The biodegradable polymers are lactide/glycolide copolymer (PLGA), polylactic acid (PLA), polyglycolic acid (PGA), polycaprolactone (PCL), lactide/caprolactone copolymer (PLC), poly (L-lactide) (PLLA), and mixtures, copolymers, or block copolymers thereof.

In a particular embodiment, the biodegradable polymer is PLGA. In one embodiment, the molar ratio of lactic acid to glycolic acid in the PLGA is 90% lactic acid to 10% glycolic acid, 85% lactic acid to 15% glycolic acid, 75% lactic acid to 25% glycolic acid, 65% lactic acid to 35% glycolic acid, 50% lactic acid to 50% glycolic acid, 35% lactic acid to 65% glycolic acid, 25% lactic acid to 75% glycolic acid, 15% lactic acid to 85% glycolic acid, and 10% lactic acid to 90% glycolic acid.

In another particular embodiment, the biodegradable polymer is PCL, PLC, PLLA, or mixtures, copolymers, or block copolymers thereof.

### Compositions and Implants

In one embodiment, the compositions of the invention comprise combinations of photopolymerizable compositions, and biodegradable polymers, as claimed, in combination with a photoinitiator and a therapeutic agent, which can be delivered to the eye to achieve controlled drug delivery to treat a range of eye diseases. The injectable ocular compositions of the invention include:
i) compositions which can be injected into the eye followed by application of short-term UV light to induce *in situ* photocrosslinking, resulting in implant formation, termed as *in situ* photocrosslinked implants (ISPcl); and
ii) compositions which can be photocrosslinked prior to application in the eye to form an implant of desired shape and size (e.g. film, rod or nano/microparticles) that can be administered intraocularly to provide a desired period of drug delivery, termed as pre-formed photocrosslinked implants (PPcl).

Alternatively, the compositions of the invention can be used to coat ocular devices, including both *in situ and* pre-formed ocular devices.

The implants of the present invention can be of any desired shape and size, such as but not limited to for example rectangular, square, cylindrical, circular, oval, films, dumbbell, rods, beads, etc., as, for example, macro, micro or nanoparticles.

The ocular implant can be less than about 10 mm, or less than about 5 mm. For instance, the ocular implant can be a rectangular implant of dimensions 10 x 5 x 0.5 mm.

In one embodiment of the present invention, the ocular injectable composition is a nanoparticle or a microparticle ocular implant.

In one embodiment, the nanoparticle ocular implant is less than about 1,000 nm, less than about 900 nm, less than about 750 nm, less than about 500 nm, or less than about 100 nm.

In one embodiment, the microparticle ocular implant is less than about 1,000 µm, less than about 900 µm, less than about 750 µm, less than about 500 µm, or less than about 25 µm.

### In situ photocrosslinked implants (ISPcl)

*In situ* photocrosslinked implants (ISPcl) of the present invention are those that form and take up their final localised structure once they are inserted into the body. The ability of these implants to fill irregular defects is an advantage of ISPcls. The ISPcls of the present invention also have additional advantages, which include, site-specific action due to relatively easy and less invasive application, localized delivery to specific tissues, prolonged delivery times, reduction in side effects linked with systemic delivery and also superior patient comfort and compliance. Additional advantages of the ISPcls of the present invention include, not requiring extreme pH conditions or elevated temperatures during processing, which could cause issue when working with temperature or pH labile drugs (e.g. proteins, peptides or genetic material). Furthermore, rapid crosslinking at physiological temperatures can swiftly entrap drug molecules and can result in an ISPcl that possesses the exact required dimensions for controlled drug release. Photocrosslinking is also beneficial in comparison to spontaneous crosslinking (e.g. enzymatic, self-assembled, Michael addition) as the initiation of the process is only triggered when exposed to a light source, therefore premature gelation is not an issue resulting in excellent control of material formation. Furthermore, short-term application of UV light will not cause any safety issues as it is considered safe for ocular applications, as UV light is clinically used for corneal crosslinking. Importantly, administration by this method allows the injection of a relatively low viscosity material into the body, which then solidifies to form a semi-solid depot that controls the drug delivery to provide short or long-term therapeutic action.

In one embodiment, the ISPcls of the present invention are formed by injection of a composition of the invention into a subject in need thereof and subsequent crosslinking using external source of UV light that results in formation of a solid implant which controls drug release for a desired period of time

For ISPcls of the invention the molecular weight of the photopolymerizable composition is typically between 200 and about 6,000 Da, between 200 and about 3,000 Da, or between 200 and 1,000 Da.

### Pre-formed photocrosslinked implants (PPcl)

The pre-formed photocrosslinked implant (PPcl) can be inserted in the eye (e.g. in the fornix, subconjunctively, intracameral intrastromal/intracorneal, transsclerally/periocular, intrasclerally or intravitreally) to treat the front of the eye or back of the eye diseases. These implants can be fabricated in a variety of shapes (e.g. rods, films, cylindrical or circular) and sizes, including in the form of micro or nanoparticles.

The PPcls of the present invention have the advantage of high crosslink density and/or a tight polymer network structure which can be configured to control drug release and/or eliminate any burst release.

The PPcls of the present invention can be fabricated to have a single and/or multiple layers, which will enable loading of more than one drug or the same drug with different release profiles or rates. Furthermore, the rate of degradation of the implants can be slower for PPcls when compared to ISPcls of the invention and can be altered to treat specific diseases or disorders.

For the PPcls of the invention the molecular weight of the photopolymerizable polymers is in the range of 200 to 20,000 Da, or in the range of 200 to about 10,000 Da.

In one embodiment, the biodegradable polymer is essentially contained within a matrix of the photopolymerizable composition. In one embodiment, the biodegradable polymer is essentially contained within a matrix of the photopolymerizable composition that forms a gel upon mixing. In one embodiment the photopolymerizable polymer is crosslinked in presence of a photoinitiator and the biodegradable polymer and therapeutic agent(s). In one embodiment, the biodegradable polymer is essentially trapped within the crosslinked photopolymerizable polymer matrix, and the therapeutic agent(s) are either dispersed or dissolved between the two phases (i.e., photopolymerizable and/or biodegradable polymer). In one embodiment, the biodegradable polymer is hydrophobic in nature and the photopolymerizable polymer is hydrophilic in nature. In one embodiment, the degree of crosslinking of the composite implant will govern the rate and extent of release of the therapeutic agent(s).

In one embodiment, the present invention is an ocular composition comprising:
i) 99 to 60 % (w/w) of a photopolymerizable composition selected from the group consisting of polyalkylene glycol diacrylate and polyalkylene glycol dimethacrylate, wherein the photopolymerizable composition has a molecular weight in the range of 200 to 20,000 Dalton;
ii) a biodegradable polymer selected from the group consisting of polyglycolides, polylactides, polycaprolactones, and mixtures, copolymers, and block copolymers thereof;
iii) a photoinitiator; and
iv) a therapeutic agent.

In one embodiment, the photopolymerizable composition is 96.9 % (w/w) and the biodegradable polymer is 2.5 % (w/w), the photopolymerizable composition is 94.1 % (w/w) and the biodegradable polymer is 5 % (w/w), the photopolymerizable composition is 69.4 % (w/w) and the biodegradable polymer is 30 % (w/w).

In another embodiment, the present invention is an ocular composition wherein i) and ii) are:
i) 79.5 to 59.5 % (w/w) polyethylene glycol diacrylate or polyethylenene glycol dimethacrylate; and
ii) 20 to 40 % (w/w) PLGA, wherein the molar ratio of lactic acid to glycolic acid in the PLGA is 90% lactic acid to 10% glycolic acid, 85% lactic acid to 15% glycolic acid, 75% lactic acid to 25% glycolic acid, or 50% lactic acid to 50% glycolic acid.

In another embodiment, the present invention is an ocular composition wherein i) and ii) are:
i) 79.5 % (w/w) polyethylene glycol diacrylate or polyethylene glycol dimethacrylate; and
ii) 30 % (w/w) PLGA wherein the molar ratio of lactic acid to glycolic acid in the PLGA is 90% lactic acid to 10% glycolic acid, 85% lactic acid to 15% glycolic acid, 75% lactic acid to 25% glycolic acid, or 50% lactic acid to 50% glycolic acid.

In another embodiment, the present invention is an ocular composition wherein i) and ii) are:
i) 95.5 to 84.5 % (w/w) polyalkylene glycol diacrylate or polyalkylene glycol dimethacrylate; and
ii) 4 to 15 % (w/w) PCL.

In another embodiment, the present invention is an ocular composition wherein i) and ii) are:
i) 69.5 to 94.5 % (w/w) polyalkylene glycol diacrylate or polyalkylene glycol dimethacrylate; and
ii) 20 to 5 % (w/w) PLLA.

In yet another embodiment, the present invention is an ocular composition wherein i) and ii) are:
i) 95.5 to 84.5 % (w/w) polyalkylene glycol diacrylate or polyalkylene glycol dimethacrylate; and
ii) 4 to 15 % (w/w) PLC in which lactic acid to caprolactone is in the range of 90 % lactic acid to 10 % caprolactone, 80 % lactic acid to 20 % caprolactone, 70 % lactic acid to 30 % caprolactone, 60 % lactic acid to 40 % caprolactone, or 50 % lactic acid to 50 % caprolactone.

In one embodiment, the present invention is an ocular composition wherein i) is 95.5 to 84.5 % (w/w) polyalkylene glycol diacrylate or polyalkylene glycol dimethacrylate; and wherein ii) is 4 to 15 % (w/w) PCL.

In one embodiment, the % of the biodegradable polymer is 30% w/w, 5% w/w, 2.5 % w/w, between 4-10% w/w, or between 5-18% w/w.

In one embodiment, i) and ii) of the compositions of the present invention are PEGDA and PLGA.

### PLGA

PLGA is prepared by polymerisation of lactic acid and glycolic acid monomers. The glass transition temperatures (T_{g}) of PLGA copolymers are above physiological temperatures of 37 °C, which imparts a moderately rigid chain configuration and therefore the mechanical strength at ambient temperatures. The use of PLGA in different lactide (LA) to glycolide (GA) ratio and molecular weight allows different drug release profiles. An increase in GA content will result in an increased water uptake of PLGA and therefore more rapid degradation. The degradation of PLGA with LA/GA 50/50 is typically between about 1 and about 3 months.

### PEGDA

PEGDA is a synthetic polymer, available in different M_{w}. PEGDA is extremely amenable to mechanical, structural and chemical alteration and so resulting in hydrogels with variable properties in terms of drug delivery and other biomedical applications. PEGDA is formed through the functionalization of the end of each PEG molecule with an acrylate group. PEGDA is also non-toxic, eliciting only a minimal immunogenic response. PEGDA has double-bond containing acrylate end groups which show rapid polymerisation when exposed to light in the presence of an appropriate initiator to produce a hydrogel network.

In one embodiment, the present invention is a PLGA/PEGDA PPcl.

In one embodiment, the present invention is a PLGA/PEGDA ISPcl.

### Copolymers

All of the copolymers and block copolymers as claimed can be used in any of the compositions and implants of the invention in combination with any of the claimed photopolymerizable compositions and biodegradable polymers, and with any therapeutic agents, photoinitiators, solvents, co-solvents, pore forming agents, and co-initiators described herein.

As used herein "copolymer" is a mixture of two or more different types of monomer units. As used herein "block copolymer" is a mixture of two or more homopolymer subunits.

In one embodiment, block or copolymers with PGA, PCL, PLA and PLGA would include any other polymeric component of the polymer e.g. PEG and PEO, for example, PLGA-PEO, PCL-PEO and PEG-PLGA, PEG-PCL block copolymers, which include, for example, PEO-PLGA-PEO, PLGA-PEG, PLGA-PEO, and PLGA-PEO-PLGA.

### Solvents

All of the solvents described herein can be used in the preparation of any of the compositions and implants of the invention in combination with any of the claimed photopotymerizabte compositions and biodegradable polymers, and with any therapeutic agents, photoinitiators, pore forming agents, and co-initiators described herein.

In one embodiment, the co-solvents used in the present invention can be selected from dichloromethane, tetrahydrofuran, ethyl acetate, acetone, dimethylformamide, acetonitrile, acetic acid, methanol, ethanol, isopropanol, glycofurol or butanol.

In one embodiment, the solvents used in the present invention are dimethyl sulfoxide, decylmethyl sulfoxide, 2-pyrrolidone, *N*-vinyl-2-pyrrolidone, 1-methyl-2-pyrrolidone, N-Methyl-2-pyrrolidone, N-ethyl-pyrrolidone, glycerol formal, glycerol, polyethylene glycol, propylene glycol, benzyl alcohol, benzyl benzoate, ethyl benzoate, triacetin, triethyl citrate, dimethylformamide, dimethylacetamide or tetrahydrofuran.

In another embodiment, the solvent is dimethyl sulfoxide, decylmethyl sulfoxide, 2-pyrrolidone, 1-methyl-2-pyrrolidone, N-Methyl-2-pyrrolidone, or glycerol formal.

In one embodiment, a solvent is used when the biodegradable polymer is PCL, PLC and/or PLLA. In one embodiment the solvent is N-Methyl-2-pyrrolidone and N-Vinyl-2-pyrrolidine when the biodegradable polymer is PCL, PLC and/or PLLA. In another embodiment, a solvent is used when the photopolymerizable composition is PEGDA.

### Pore forming agents

In one embodiment, a suitable pore forming agent may be selected in view of the specific therapeutic agent and composition of the implant, and the desired elution profile or release rate. The pore forming agent may be a naturally occurring agent or polymer or a synthetic agent or polymer.

In another embodiment, implant porosity can be adjusted by preparing implants in the presence of dispersed water-soluble porosigens, which can be removed later by washing with water to leave an interconnected meshwork (i.e., porous hydrogels). The pore size of hydrogels prepared by the porosigen technique depends on the size of the porosigens.

All of the pore forming agents described herein can be used in any of the implants and compositions of the invention in combination with any of the claimed photopolymerizable compositions and biodegradable polymers, and with any therapeutic agents, photoinitiators, solvents, co-solvents, and co-initiators described herein.

In one embodiment, the compositions of the invention further comprise a pore-forming agent.

In one embodiment, the pore-forming agent is polyethylene glycol, lactose, maltose, glucose, mannitol, gelatin, sodium chloride, magnesium carbonate, magnesium hydroxide, potassium chloride, sodium bicarbonate, ammonium bicarbonate, potassium bicarbonate, chitosan, polyvinylpyrrolidone, polyvinyl alcohol, agarose or sucrose.

### Photoinitiators

In one embodiment, the compositions of the invention further comprise a photoinitiator.

The photoinitiators described herein can be used in any of the compositions and implants of the present invention in combination with any of the claimed photopolymerizable compositions and biodegradable polymers, and with any therapeutic agents, photoinitiators, solvents, co-solvents, and co-initiators described herein.

In certain embodiments, the photoinitiator is designed to work using light from about 200 to about 550 nm. In some embodiments, a photoinitiator is designed to work using UV light from about 200 to about 400 nm.

In certain embodiments, the light source may allow variation of the wavelength of light and/or the intensity of the light. Light sources useful in the present invention include, but are not limited to, lamps, fiber optics devices, etc.

In one embodiment, the photoinitiator is a ketone (e.g., RCOR'). In one embodiment, the compound is an azo compound (e.g., compounds with a -N=N- group). In one embodiment, the photoinitiator is an acylphosphineoxide. In one embodiment, the photoinitiator is a sulfur-containing compound. In one embodiment, the initiator is a quinone. In certain embodiments, a combination of photoinitiators is used.

In one embodiment, the photoinitiator is a hydroxyketone photoinitiator, an amino ketone photoinitiator, a hydroxy ketone/benzophenone photoinitiator, a benzyldimethyl ketal photoinitiator, an acyl phosphine oxide photoinitiator, an acyl phosphine oxide/alpha hydroxy ketone photoinitiator, a benzophenone photoinitiator, a ribityl isoalloxazine photoinitiator, or a phenylglyoxylate photoinitiator or any combination thereof.

In one embodiment the photoinitiator is 1-[4-(2-hydroxyethoxy)-phenyl]-2-hydroxy-2-methyl-1-propanone, 2,2-dimethoxy-2-phenylacetophenone (DMPA) or riboflavin.

In one embodiment, the compositions of the present invention further comprise a co-initiator. In one embodiment, the co-initiator is eosin Y, triethanolamine, camphorquinone, 1-vinyl-2 pyrrolidinone (NVP), eosin, dimethylaminobenzoate (DMAB), Irgacure^{®} 907 (Ciba Geigy), Irgacure^{®} 651 (Ciba Geigy), Darocur 2959 (Ciba Geigy), or ethyl-4-N,N-dimethylaminobenzoate (4EDMAB).

In one embodiment, the photoinitiator is riboflavin and the co-initiator is L-arginine.

### Process

The compositions and implants of the present invention can be made by any methods known in the art as well as the methods described herein. The methods described herein are applicable to all compositions and implants of the invention.

In one embodiment, polymer M_{w}, type and copolymer ratio, drug type and loading, implant size, time and extent of UV crosslinking and/or amount and type/concentration of photoinitiator and/or pore forming agent (porogen) and/or solvent/co-solvent can be altered to control the rate and extent of drug release. The alteration of these factors provides compositions of the invention that can be easily tailored to produce a desired period of drug release to address specific clinical/patient needs in treating various ocular diseases.

In one embodiment, the present invention is a method of making the ocular compositions of the present invention, the method comprising the steps of:
i) mixing a therapeutic agent with a photopolymerizable composition, a biodegradable polymer and a photoinitiator, in any order of addition;
ii) irradiating the mixture i) with light at a wavelength of between 230 to 550 nm between 300 to 525 nm, or between 350 to 490 nm for between 1 second and 60 minutes to form the ocular composition; and
wherein the composition ii) is for administration to an ocular area of a subject.

In another embodiment, the present invention is a method of making the ocular composition of the present invention, the method comprising the steps of:
i) mixing a therapeutic agent with a photopolymerizable composition;
ii) adding a biodegradable polymer to the mixture i)
iii) adding a photoinitiator to the mixture ii);
iv) irradiating the mixture iii) with light at a wavelength of between 230 to 550 nm between 300 to 525 nm, or between 350 to 490 nm for between 1 second and 60 minutes to form the ocular composition; and
wherein the composition iv) is for administration to an ocular area of a subject.

In the above embodiments, the irradiating step is with light at a wavelength of about 365 nm or about 475 nm for 1 second, about 2 minutes, about 5 minutes, about 10 minutes, about 20 minutes or about 30 minutes.

In another embodiment, the present invention is a method of making the nanoparticle or microparticle ocular implant, comprising the steps of:
i) mixing the therapeutic agent, the photopolymerizable composition, the biodegradable polymer and the photoinitiator, in any order of addition, to form mixture i);
ii) adding the mixture i) to an aqueous medium to form mixture ii);
iii) sonicating the mixture ii); and irradiating the mixture ii) with light at a wavelength of between 230 to 550 nm, between 300 to 525 nm, or between 350 to 490 nm for between 1 second and 60 minutes to form the nanoparticles or microparticles.

In another embodiment, the present invention is a method of making the nanoparticle or microparticle ocular implants of the present invention, comprising the steps of:
i) mixing a therapeutic agent with a photopolymerizable composition;
ii) adding a biodegradable polymer to the mixture i)
iii) adding a photoinitiator to the mixture ii);
iv) adding the mixture iii) to a aqueous medium;
v) sonicating the mixture iv); and
vi) irradiating the mixture v) with light at a wavelength of between 230 to 550 nm between 300 to 525 nm, or between 350 to 490 nm for between 1 second and 60 minutes to form the nanoparticles or microparticles.

In one embodiment the aqueous medium is a combination of water and phosphate buffered saline (PBS).

In one embodiment, step iv) is carried out in a step-by-step manner to emulsify the mixture. As used herein step-by-step means that the mixture is not added all at once, but rather it is added in stages with breaks of between the additions.

In another embodiment, i) the therapeutic agent is mixed with a portion of photopolymerizable composition and ii) another portion of photopolymerizable composition is mixed with biodegradable polymer, then iii) the two portions are mixed together. The mixture iii) is added to a aqueous medium, and then sonicated. Finally the mixture is irradiated with light at a wavelength of between 230 to 550 nm, between 300 to 525 nm, or between 350 to 490 nm for between 1 second and 60 minutes to form the nanoparticles or microparticles Irradiation can be applied during sonication i.e. sonicating the mixture under UV light, in other words, the aqueous medium will be under UV light (at defined wavelength) and sonication, followed by step-by-step addition of the mixture.

In another embodiment, the sonication time, gel composition, phase ratio (of the gel vs aqueous medium), and rate of adding the gel mixture into aqueous medium are adjusted to form a nanoparticle or microparticle.

In the compositions of the present invention, varying the UV crosslinking time can control the rate of and duration of drug release. In some embodiments, an increase in UV crosslinking times causes a decrease in drug release. Additionally, varying the concentration of the photoinitiator can control the rate and duration of drug release. Furthermore, varying both the UV crosslinking time and the concentration of photoinitiator can control the rate and duration of drug release. In one embodiment, decreasing the concentration of the biodegradable polymer (such as PLGA) increases the drug release rate. In one embodiment, adding a pore-forming agent (e.g. MgCO₃), increases the drug release rate. In one embodiment, higher UV crosslinking time and higher concentration of photoinitiator can sustain the drug release for longer periods of time. In one embodiment, the drug release can be sustained for a period of greater than about 1 day, about 2 days, about 1 week, about 1 month, about 2 months, about 3 months, or about 6 months.

In one embodiment, the duration of drug release in the ISPcls of the present invention can be considerably extended, for example, providing controlled drug release for a period of greater than 200 days (>6 months). This duration can be varied by varying the degree of crosslinking.

In some embodiments, the slow degradation rate of the ISPcls of the present invention provide protection of the sensitive molecules such as peptides and proteins. It has been shown below, that the ISPcls of the present invention are stable and avoid protein degradation and maintain protein activity.

In some embodiments, burst release can be eliminated or controlled by varying the UV crosslinking time.

In one embodiment, the present invention is a PPcl with no burst release. In one embodiment, the present invention is a PPcl with high crosslinking density that significantly slows drug diffusion.

### Methods of use

Any of the implants and compositions described herein are suitable for use in any of the methods described herein.

In one embodiment of the present invention, the injectable ocular compositions can be for use in a method of treating a disease or disorder of the eye in a subject in need thereof, comprising administering a composition or implant of the present invention to an ocular area of the subject.

In one embodiment, the present invention is a composition or implant of the present invention for use in treating a disease or disorder of the eye in a subject in need thereof.

As used herein, an "ocular area" is an area inside, outside or adjacent to the eye of the subject. In one embodiment, the ocular area is the sclera (intrascleral), outside the sclera (transscleral), the vitreous body, the choroid, the cornea, the stroma, intracameral, the aqueous humor, the lens, the fornix, or the optic nerve.

The compositions are administered by injection, including, intravitreal, subconjunctival, peribulbar, subtenon or retrobulbar injections and cornea. The implants can be administered in the same way.

The implants can also be administered via a surgical procedure. In some embodiments, the implants are secured in place, after surgical implantation, via an adhesive or sutures.

The term "subject" refers to an animal (e.g., a bird such as a chicken, quail or turkey, or a mammal), specifically a "mammal" including a non-primate (e.g., a cow, pig, horse, sheep, rabbit, guinea pig, rat, cat, dog, and mouse) and a primate (e.g., a monkey, chimpanzee and a human), and more specifically a human. In one embodiment, the subject is a non-human animal such as a farm animal (e.g., a horse, cow, pig or sheep), or a pet (e.g., a dog, cat, guinea pig or rabbit). In another embodiment, the subject is a "human".

As used herein, the terms "treat", "treatment" and "treating" refer to therapeutic treatments that include the reduction or amelioration of the progression, severity and/or duration of a disease, disorder or condition, or the amelioration of one or more symptoms (specifically, one or more discernible symptoms) of a disease, disorder or condition, resulting from the administration of the compositions or implant of the invention. In specific embodiments, the therapeutic treatment includes the amelioration of at least one measurable physical parameter of a disease, disorder or condition. In other embodiments the therapeutic treatment includes the inhibition of the progression of a condition, either physically by, e.g., stabilization of a discernible symptom, physiologically by, e.g., stabilization of a physical parameter, or both. In other embodiments the therapeutic treatment includes the reduction or stabilization of a disease, disorder or condition.

Exemplary therapeutic agents include, but are not limited to, polypeptides, nucleic acids, such as DNA, RNA, and siRNA, growth factors, steroid agents, antibody therapies, antimicrobial agents, antibiotics, antiretroviral drugs, anti-inflammatory compounds, antitumor agents, anti-angiogeneic agents, and chemotherapeutic agents.

In one embodiment, the therapeutic agent of the present invention includes, but is not limited to, ketorolac, naphazoline, lidocaine, bevacizumab, aflibercept, pegaptanib, brimonidine, dorzolamide, azithromycin, rapamycin, bepotastine besilate, diclofenac, besifloxacin, cysteamine hydrochloride, fluocinolone acetonide, difluprednate, tasimelteon, ocriplasmin, enoxaparin sodium ranibizumab, latanoprost, timolol, bimatoprost, ofloxacin, cephazolin, phenylephrine dexamethasone, triamcinolone acetonide, levofloxacin, cyclophosphamide, melphalan cyclosporine, methotrexate, azathioprine, , travoprost, verteporfin, tafluprost, ketotifen fumarate, foscarnet, amphotericin B, fluconazole, voriconazole, ganciclovir, acyclovir, gatifloxacin, vitamin (vitamin A, vitamin C, and vitamin E), zinc, copper, lutein, zeaxanthin or combinations thereof.

In one embodiment, the compositions or implants of the present invention can deliver a bioactive agent a large molecular weight drug, such as, aflibercept, pegaptanib, or an antibody therapeutic, such as ranibizumab, bevacizumab, trastuzumab, rituximab, gentuzumab, ozagamicin or cetuximab. In some embodiments, the M_{W} of the therapeutic agent is greater than about 10 kDa, about 30 kDa, about 50 kDa, about 75 kDa, about 100 kDa, about 150 kDa, about 200 kDa.

The disease, or disorder can be pain, inflammation, cataracts, allergies, age-related macular degeneration (AMD), diabetic retinopathy (DR), macular edema, diabetic macular edema (DME), cytomegalovirus (CMV), retinitis, retinitis pigmentosa, uveitis, dry-eye syndrome, keratitis, glaucoma, blepharitis, blephariconjunctivtis, ocular hypertension, conjunctivitis, cystinosis, vitreomacular adhesion, corneal neovascularisation, corneal ulcers and post-surgical ocular inflammations/wound healing.

### EXAMPLES

### Materials

The following methods and materials were used in the Examples below.

Poly(lactic-co-glycolic acid) (PLGA) 5002A (50% lactic acid, 50% glycolic acid monomers) and PLGA 7502A (75% lactic acid, 25% glycolic acid) (referred to as PLGA50/50 and PLGA75/25 respectively throughout) were purchased from Corbion Purac Biomaterials (Gorinchem, The Netherlands).

Poly(ethylene glycol) diacrylate (PEGDA) molecular weight (M_{w}) 258, 575 and 700 Da, ovalbumin (OVA), bovine serum albumin (BSA), Irgacure 2959 (1-[4-(2-hydroxyethoxy)-phenyl]-2-hydroxy-2-methyl-1-propanone), methanol (HPLC grade) and acetonitrile (ACN) (HPLC grade) were purchased from Sigma-Aldrich (Dorset, United Kingdom). Triamcinolone acetonide (TA) and dexamethasone (DEX) were purchased from Spruyt Hillen bv (Ijsselstein, The Netherlands). Bevacizumab (BVZ) (Avastin^{®}) was purchased from local pharmacy (manufactured by Roche, Switzerland; each vial consists of 100 mg BVZ in 4 mL i.e, 25mg/ml). Fluorescein isothiocyanate-dextran (FITC-Dextran) (M_{w} 150kDa) was purchased from TdB Consultancy AB (Uppsala, Sweden). 27G needles and 1ml syringes were purchased from Terumo Europe N.V. (Interleuvenlaan, Belgium). Rabbit anti-OVA-biotin conjugate (polyclonal) was purchased from Novus Biologicals (Cambridge, United Kingdom). Streptavidin-Horse radish peroxidase conjugate was purchased from BioLegend^{®} (San Diego, United States). Superblock T20 buffer was purchased from Thermo Scientific Pierce (Rockford, United States).

In the following examples, only the injectable ocular compositions as claimed are according to the invention.

### In situ photocrosslinked implants (ISPcl)

### Example 1, Preparation of ISPcl gel formulations

For preparation of ISPcl, the molecules under investigation (DEX, OVA, BSA, FITC-dextran 150kDa and BVZ) were firstly added in selected PEGDA (M_{w} = 700 Da) at a concentration of 0.5% w/w. Once the molecules were fully dissolved or suspended, the desired amount of PLGA 75/25 was then added to the molecule/PEGDA mixture and left to dissolve at room temperature to produce 30% w/w PLGA formulations. Prior to photocrosslinking predetermined amount of photoinitiator Irgacure^{®} 2959 (2% w/v in 70% ethanol in water as stock solution) was added to the formulation and vortexed for predetermined time to ensure complete mixing.

### Example 2, In vitro drug release study

For drug release studies selected ISPcl gel formulation were injected (approx. 0.2 g or 0.1 g) inti a desired amount of PBS (pH 7.3± 0.2). Sink conditions were maintained for each drug type. ISPcl were formed by exposing them immediately to 365 nm using bench-top UV light (at 3.1 ± 0.1 mW/cm², CAMAG, Muttenz, Switzerland) for varying periods of time in PBS. The implants were stored in an incubator (37°C and 60 rpm) for the duration of the release study. At predetermined time intervals the entire PBS medium was removed and replaced with equal amount of fresh PBS. All the experiments were carried out in triplicates. The concentration of released drug molecule in the PBS samples was analyzed as described below.

Analysis of DEX and TA samples were carried out using reversed-phase HPLC with UV detection (Agilent 1260 Infinity Quaternary System) using an Agilent Zorbax Eclipse Plus 250mm C18 column (250 mm length, 4.6 mm internal diameter and 5 µm particle size) and an Agilent Zorbax guard column held at 25°C (Agilent Technologies UK Ltd, Stockport, UK). Analysis required a mobile phase of 60% water and 40% acetonitrile with UV absorbance at 270 nm (for DEX) and 236 nm (for TA) at a flow rate of 1 ml/min and 0.8 ml/min respectively. Analysis of FITC-Dextran 150kDa release was conducted using a fluorescence spectrophotometer. To a black 96-well plate, 150 µL of FITC-dextran 150kDa sample was pipetted. The plate was then analysed using a BMG Labtech FLUOstar Optima fluorescence plate reader (BMG Labtech GMBH, Ortenberg, Germany). Fluorescence excitation occurred at 480 nm with emission measured at 520 nm. The gain was set at 828 and the plate was read at 37°C. Fluorescence values were collected and examined using BMG Labtech OPTIMA software (version 2.20). BSA, OVA and bevacizumab (Avastin) were assayed using a Pierce^{™} Micro BCA protein assay kit (Thermo Scientific, Hampton, UK). To a microwell, 150 µL of BSA, OVA or BVZ standard or release sample was pipetted and 150 µL of working reagent was added. A plate shaker ensured thorough mixing. The 96-well plate was covered and incubated for 2 hours at 37°C. Once the plate was allowed to return to room temperature, a UV plate reader measured the absorbance at 562 nm. The average absorbance reading of the blank was subtracted from those of the standards/ samples. ELISA test for determining the bioactivity of OVA was tested as per our in house protocol. Figure 1A clearly demonstrates that the ISPcl forms an implant when injected into aqueous environment and subjected to UV light. Figure 1B indicates that the implants degrade over time.

There are numerous factors that govern the extent and rate of drug release and/or biodegradation of the implants, for example, polymeric composition, polymer M_{w}, drug type and loading, implant size, time and extent of UV crosslinking and amount and type/concentration of photoinitiator will determine the rate and extent of drug release. The ability to vary these factors also means that implants can be easily tailored to produce a desired period of drug release to address specific clinical/patient needs in treating various ocular diseases, this is clearly demonstrated in Figures 2-5.

Figures 2-5 demonstrate *in vitro* release profiles of various drug molecules from ISPcl of different compositions and crosslinking times. Fig. 2 shows that the percentage release of DEX is dependent upon the time of UV crosslinking, where increase in crosslinking time caused decrease in percentage drug release. For example, after nearly 140 days the mean percentage DEX release was 79.62, 75.15, 69.59 and 64.21 from ISPcl crosslinked for 5, 10, 15 and 30 min, respectively. In all cases low burst release (<15%) is noted, which is also dependent upon the time of UV crosslinking. In addition to small molecules, the mainstay of treatment of PS diseases such as AMD, DR and DME treatment are anti-VEGF therapies such as bevacizumab (Avastin^{®}) and ranibizumab (Lucentis^{®}), it was paramount to investigate the ability of these ISPcls to deliver large molecular weight biologics molecules for long-term, as these drugs are injected indefinitely in the eye on monthly basis. Therefore, we have investigated release of model protein molecule, BSA and OVA and anti-VEGF molecule bevacizumab (BVZ). BSA has a M_{W} of 66kDa. OVA has a M_{W} of 45kDa which is nearly similar in M_{w} to commercially available anti-VEGF drug ranibizumab (Lucentis^{®}). BVZ has a high M_{W} of 149kDa. Figure 3 shows long-term controlled release of BSA from 30%PLGA/69.4% w/w PEGDA700 crosslinked implants for 5 min, with nearly 86% of BSA released after 200 days. Likewise, Fig. 4 and 5 shows controlled release of BVZ and OVA from the ISPcl implants. It is evident that either by varying the concentration of the photoinitiator or crosslinking time the amount of drug released can be controlled, where higher crosslinking time or higher concentration of photointiator can sustain the drug release for longer periods of time. For example, approximately 41% and 100% BVZ was released from 30%PLGA ISPcl implants UV crosslinked for 2.5 min and 30 sec, respectively. Similar trend was noted with regards to OVA release from ISPcl. It is clear from the Figures 4 and 5, that the drug release can be sustained for a period >140 days, by varying the crosslinking time.

Furthermore, unlike PLGA only implants, which have a short degradation time (e.g. 50-60 days for PLGA 50/50 and 3-4 months for PLGA 75/25 alone), drug release from ISPcls can be considerably extended due to the cross-linked nature of the implant - where controlled drug release for a period of greater than 200 days (>6 months) has been demonstrated (Fig. 2-3), which can be varied by varying the degree of crosslinking.

Due to slow degradation rate of the crosslinked ISPcIs, the drastic drop in local pH is further delayed (unlike PLGA only implants) which is especially important and an advantage in protecting the sensitive molecules such as peptides and proteins. We have demonstrated that our novel ISPcl systems are stable and avoid protein degradation. For example, an ELISA test was conducted to determine the bioactivity of released OVA from the ISPcl, at 37±2°C, after 1 and 3 months, respectively. Nearly 97±2% of the OVA remained active as demonstrated by the ELISA, which clearly indicates excellent stability of protein molecules in our delivery systems. This clearly indicates that the PLGA/PEGDA implants not only sustain the release of protein molecules but they also maintain protein activity.

### Example 3, Syringeability of ISPcl

Syringeability is a very important parameter in considering whether a formulation is suitable to be delivered *via* a syringe and needle, especially if the needle in question has a small bore, as would be required for ocular delivery. Therefore, Work of Syringeability (WoS) was investigated to determine the effort that would be required to expel the ISPcl gel formulations through 27G needle that is commonly used in intraocular injections. Briefly, 1ml disposable medical syringes (Becton, Dickinson and Company, Oxford, UK) were filled with the ISPcl gel formulations to a constant height equivalent to 0.1 ml. Using the Texture Analyser (Stable Micro Systems, Surrey, UK), the content of the syringe was expelled at a rate of 0.5mm/second. The area under the resultant force-distance plot was used to determine the WoS using the Exponent TA.XT software (Version 4.0). The WoS observed relating to expelling air from a blank syringe was subtracted from the experimental results to ensure the data collected related solely to the formulation under study. An increase in WoS was conveyed by an increase in the area under the curve. All measurements were performed in at least triplicate.

In addition to drug release, it is also important to demonstrate the injectability of these *in situ* forming implant gels, as these are designed to be injected in the eye using hypodermic needles or microneedles following short-term application of UV light. Figure 6 represents the WoS for each ISPcl formulation that was calculated from the resulting force-distance plots of Texture-Analysis. The WoS data indicates that the PLGA/PEGDA formulations for both PLGA 50/50 and PLGA 75/25 require different forces to expel them from the syringe with 27G needle. In general the PLGA75/25 formulations are more easily expelled compared to the PLGA50/50 formulations with a WoS of 43.23 N.mm calculated for the PLGA50/50-PEGDA700 formulation, with 22.55 N.mm calculated for the PLGA75/25-PEGDA700 formulation. It would be expected that the highest molecular weight of PEGDA would result in the greatest resistance to expulsion. This trend is followed when considering the PLGA75/25 formulations but not with the PLGA50/50. The greatest WoS was seen with the PLGA 50/50-PEGDA258 formulation, 48.24 N.mm, which is significantly greater than the other PLGA5050 formulations (*p*< 0.0001). Therefore, the implant forming gels can be injected and the forces for injections vary by changing the composition/concentration of the polymers within the ISPcl formulation.

### Example 4, Preformed Photocrosslinked implants (PPcl)

Similar to ISPcl the molecule/drug under investigation BSA, TA, OVA, and FITC-dextran 150kDa was firstly dissolved/suspended in PEGDA at different concentrations. Next, a desired amount of PLGA 75/25 or 50/50 was added to the drug/PEGDA mixture and left for mixing at room temperature to form a homoaenous gel. Finally, a desired amount of a photoinitiator Irgacure^{®} 2959 (2% w/v in 70% ethanol in water) was added to the formulation and vortexed for 1 minute to ensure complete mixing. These gels were then casted into molds to form thin films (10 x 5 x 0.5 mm) and subjected to photocrosslinking using Fusion UV LightHammer 6 high power UV curing system (Maryland, USA) fitted with a "D" class mercury discharge bulb (270W/10nm), with a belt speed of 10m/min and at wavelength of 365 nm, at different lamp intensities (LI) and for different cycles/runs (the expose time for each run is 3.4 sec) to form PPcl. *In vitro* drug release studies were conducted as given for ISPcl in PBS. Drug samples were collected at a predetermined time intervals and analyzed using the techniques as stated above.

In addition to using the ISPcl as an injectable implant forming ocular delivery system, the PLGA/PEGDA composition invented here can also be used as preformed implants. This offers additional opportunity for our delivery systems where the preformed photocrosslinked implants (PPcl) can be simply inserted in the eye (e.g. in the fornix or subconjunctively) to treat the front of the eye diseases or can also be administered (e.g. intravitreally) in the eye, using an applicator, for treating the back of the eye diseases. The PPcl are made as detailed above, Fig. 8 shows a digital and SEM image of these implants. These implants can be fabricated in a variety of shapes (e.g. rods, films, cylindrical or circular) and sizes, including in the form of micro-or nanoparticles.

Using PPcls we have demonstrated controlled release of BSA, FITC-Dextran 150kDa, and TA over different periods of time, as shown in the Figures 9-12. FITC-Dextran 150kDa was selected, as its M_{W} is nearly similar to that of commercially available anti-VEGF drug BVZ (Avastin^{®}). As shown in Fig. 9, the percentage release was dependent upon the M_{w}, where BSA showed higher percentage of release when compared to FITC-Dextran 150kDa over the period of 266 days. For example, % release of BSA and FITC-Dextran 150kDa was approx. 72 and 27%, respectively after 266 days, which is predicted to continue for another few months. This is due to the fact that the BSA molecule is nearly 2.27-times smaller in M_{W} than FITC-Dextran 150kDa. Here, we have seen nearly zero-order release of the molecules with no burst release when compared to the ISPcIs, this is due to the high crosslink density or tight network structure of the PPcl that significantly slows drug diffusion, when compared to the ISPcIs. Furthermore, like the ISPcl, the PPcls are also biodegradable however the rate of degradation is slower as compared to ISPcl. Furthermore, the PPcls can be fabricated to have a single and/or multiple layers, which will enable loading of more than one or more drug molecules or the same drug with different release profiles or rates.

Unlike long-term drug release, drug release for short-term is also advantageous in treating certain common ocular diseases of the front of the eye (e.g. glaucoma, dry-eye syndrome, keratitis, blepharitis, and other types of bacterial/fungal inflammations) that require short-term drug delivery. In this regard Figures 10-13 show short-term release of a small molecule, TA, where the TA release can be for 2 weeks to 9 weeks. Particularly, when PPcl were fabricated at IL of 50 and 100%, with 5 runs, nearly 75 and 62% of TA was released within 35 days (Fig. 10). Likewise, when a low level of PLGA was used nearly 46% of TA was released within 14 days, which increased to 52% with decrease in PLGA concentration (Fig. 11). Furthermore, by adding a pore-forming agent (e.g. MgCO₃), the amount of drug (TA) released from the implant can be increased. Without wishing to be bound by theory it is believed that an increase in pore density of the implant will allow higher drug release (Fig. 12). On the other hand, when the M_{W} of PEGDA was varied from 700 Da to 6000 Da in the preparation of PPcIs, TA release increased from 11% to 77% after 10 days (Fig. 13). This is due to the fact that the PPcl with low M_{W} PEGDA has higher crosslink density when compared to high M_{W} PEGDA. Overall, this invention suggests that by simply varying the composition and/or time of crosslinking of the PPcls the drug release can be easily tailored, thereby providing greater degree of flexibility in designing these implants that can be utilized to address specific ocular diseases, where either short-term to long-term drug release is required.

### Example 5, Biocompatability of polymeric matrix

In order to gain cytotoxicity data of the polymer matrix utilized in the preparation of ISPcl and PPcl, the materials were exposed to human retinal epithelial cells line (ARPE-19). For this a MTT (3-(4,5-dimethylthiazol-2-yl)2,5-diphenyl tetrazolium bromide) cleavage assay was used that was originally described by Mosmann for measuring cell survival/proliferation¹². The assay detects live cells therefore this method can be used to measure cytotoxicity of materials. The MTS assay is often described as a 'one-step MTT assay' as it allows the addition of the reagent straight to the cells without the intermittent steps that are required with the MTT assay. MTS (3-(4,5- dimethylthiazol-2-yl)-5-(3-carboxymethoxyphenyl)-2-(4-sulfophenyl)-2H- tetrazolium), in the presence of phenazine methosulfate (PMS), produces a formazan product that has an absorbance maximum at 490-500 nm. The PLGA/PEGDA formulation was produced in a sterile environment with the PEGDA being filtered through a sterile 0.2µm syringe filter (VWR^{®}, International Ltd, Leicestershire, UK). The ISPcl formulation (0.1g) was injected in 5ml of DMEM/F-12 media (Gibco^{®}, Life TechnologiesTM, Paisley, UK) in autoclaved glass vials. The formulation was subjected to UV crosslinking at 365 nm, similar to in vitro release studies and at predetermined time points (1, 30 and 120 days after formation), the entire release media was collected, stored and replaced with fresh media. The collected media was then subjected to cytotoxicity studies. All treatments were performed on ARPE-19 cells seeded in 96-well plates (Nunc^{®}, Denmark) at a cellular density of 1.75x10⁴ cells/well, which were incubated at 37°C for 24 h in DMEM/F-12. The DMEM/F-12 medium was removed and replaced with 200 µl of the release media from each time point (fresh media was used as the control). Subsequently, the cells were incubated for a further 24 hours. Cell viability was determined using the cell proliferation assay where 20µl of Promega G3580 MTS assay solution (Promega Corporation, Wisconsin, USA) was added to each well. After 2 hours of incubation, the UV absorbance was determined at 490 nm.

We have also demonstrated that the ISPcls are biocompatible in nature, as presented in Figure 7. When the release samples of the implants was exposed to human retinal pigment epithelial cell lines (ARPE-19) over different time periods, it showed compatibility nearly similar to that of the control samples (culture media), therefore, indicating biocompatibility with ocular cell lines.

### Example 6, In vivo implant formation

2 µl of ISPcl gel formulation was injected by intravitreal route in the rat eye, following by UV light exposure for 2 min, fundus images were collected to locate the implant formation and image model dye release within the eye. Likewise PPcl were administered by subconjunctival route and any surface inflammation monitored by an experienced ophthalmologist.

In *in vivo* experiments we have demonstrated that the ISPcls form an implant upon injection in the eye (intravitreal route) and biodegradation over time (Fig. 14). A fluorescent molecule was used in this study to show that the drug release occurs over time and implant degradation without causing any damage to the ocular tissues, as seen by fundus imaging. This indicates that this polymeric composition is biocompatible to ocular tissues and therefore considered safe for application in the eye.

## Claims

1. An injectable ocular composition comprising:
i) 99 to 60 % (w/w) of a photopolymerizable composition selected from the group consisting of polyalkylene glycol diacrylate and polyalkylene glycol dimethacrylate, wherein the photopolymerizable composition has a molecular weight in the range of 200 to 20,000 Dalton;
ii) a biodegradable polymer selected from the group consisting of lactide/glycolide copolymer (PLGA), polylactic acid (PLA), polyglycolic acid (PGA), polycaprolactone (PCL), lactide/caprolactone copolymer (PLC), poly (L-lactide) (PLLA) and mixtures, copolymers, and block copolymers thereof;
iii) a photoinitiator; and
iv) a therapeutic agent.

2. The ocular composition of Claim 1, for use to form an ocular implant or for use to coat an ocular implant, optionally wherein the implant is an *in situ* formed ocular implant.

3. The ocular composition of Claim 2, wherein the implant is an *in situ* formed ocular implant, wherein the photopolymerizable composition has a molecular weight in the range of 200 to 20,000 Dalton, more preferably in the range of 200 to 3,000 Dalton or 200 to 1,000 Dalton.

4. The ocular composition of Claim 2, wherein the implant is a pre-formed ocular implant.

5. The ocular composition of Claim 1, wherein the biodegradable polymer is PLGA or wherein the biodegradable polymer is selected from the group PCL, PLC, PLLA, and mixtures, copolymers, and block copolymers thereof.

6. The ocular composition of any one of Claims 1-5, wherein the photopolymerizable composition is polyalkylene glycol diacrylate or wherein the photopolymerizable composition is selected from the group consisting of polyethylene glycol diacrylate, diethylene glycol diacrylate, polyethylene glycol dimethacrylate, diethylene glycol dimethacrylate, polypropylene glycol diacrylate, dipropylene glycol diacrylate, dipropylene glycol dimethacrylate, and polypropylene glycol dimethacrylate; or, wherein the photopolymerizable composition is polyethylene glycol diacrylate or polyethylene glycol dimethacrylate; or wherein the photopolymerizable composition is polyethylene glycol diacrylate.

7. The ocular composition of Claim 5, wherein the molar ratio of lactic acid to glycolic acid in the PLGA is 90% lactic acid to 10% glycolic acid, 85% lactic acid to 15% glycolic acid, 75% lactic acid to 25% glycolic acid, 65% lactic acid to 35% glycolic acid, 50% lactic acid to 50% glycolic acid, 35% lactic acid to 65% glycolic acid, 25% lactic acid to 75% glycolic acid, 15% lactic acid to 85% glycolic acid, and 10% lactic acid to 90% glycolic acid.

8. The ocular composition of Claim 5, comprising:
i) 79.5 to 59.5 % (w/w) polyethylene glycol diacrylate or polyethylenene glycol dimethacrylate; and
ii) 20 to 40 % (w/w) PLGA, wherein the molar ratio of lactic acid to glycolic acid in the PLGA is 90% lactic acid to 10% glycolic acid, 85% lactic acid to 15% glycolic acid, 75% lactic acid to 25% glycolic acid, or 50% lactic acid to 50% glycolic acid; or
i) 69.5 % (w/w) polyethylene glycol diacrylate or polyethylene glycol dimethacrylate; and
ii) 30 % (w/w) PLGA wherein the molar ratio of lactic acid to glycolic acid in the PLGA is 90% lactic acid to 10% glycolic acid, 85% lactic acid to 15% glycolic acid, 75% lactic acid to 25% glycolic acid, or 50% lactic acid to 50% glycolic acid; or
i) 95.5 to 84.5 % (w/w) polyalkylene glycol diacrylate or polyalkylene glycol dimethacrylate; and
ii) 4 to 15 % (w/w) PCL; or
i) 79.5 to 94.5 % (w/w) polyalkylene glycol diacrylate or polyalkylene glycol dimethacrylate; and
ii) 5 to 20 % (w/w) PLLA.; or
i) 95.5 to 84.5 % (w/w) polyalkylene glycol diacrylate or polyalkylene glycol dimethacrylate; and
ii) 4 to 15 % (w/w) PLC in which lactic acid to caprolactone is in the range of 90 % lactic acid to 10% caprolactone, 80 % lactic acid to 20 % caprolactone, 70 % lactic acid to 30 % caprolactone, 60 % lactic acid to 40 % caprolactone, or 50 % lactic acid to 50 % caprolactone.

9. The ocular composition of any one of Claims 1-8, further comprising a solvent selected from dimethyl sulfoxide, decylmethyl sulfoxide, 2-pyrrolidone, 1-methyl-2-pyrrolidone, N-vinyl-pyrrolidine, N-Methyl-2-pyrrolidone, N-ethyl-pyrrolidone, glycerol formal, glycerol, polyethylene glycol, propylene glycol, benzyl alcohol, benzyl benzoate, ethyl benzoate, triacetin, triethyl citrate, dimethylformamide, dimethylacetamide and tetrahydrofuran; preferably wherein the solvent is selected from dimethyl sulfoxide, decylmethyl sulfoxide, 2-pyrrolidone, 1-methyl-2-pyrrolidone, N-Methyl-2-pyrrolidone, and glycerol formal.

10. The ocular composition of any one of Claims 1-9, further comprising a pore-forming agent; preferably wherein the pore-forming agent is selected from polyethylene glycol, maltose, glucose, agarose, mannitol, gelatin, sodium chloride, magnesium carbonate, magnesium hydroxide, potassium chloride, sodium bicarbonate, potassium bicarbonate, and sucrose.

11. The ocular composition of any one of Claims 1-10, wherein the biodegradable polymer is essentially contained within a matrix of the photopolymerizable composition.

12. The ocular composition of any one of Claims 1-11, wherein the photoinitiator is a hydroxyketone photoinitiator, an amino ketone photoinitiator, a hydroxy ketone/benzophenone photoinitiator, a benzyldimethyl ketal photoinitiator, an acyl phosphine oxide photoinitiator, an acyl phosphine oxide/alpha hydroxy ketone photoinitiator, a benzophenone photoinitiator, a ribityl isoalloxazine photoinitiator, or a phenylglyoxylate photoinitiator or any combination thereof; more preferably wherein the photoinitiator is 1-[4-(2-hydroxyethoxy)-phenyl]-2-hydroxy-2-methyl-1- propanone, 2,2-dimethoxy-2-phenylacetophenone (DMPA) or riboflavin.

13. The ocular composition of any one of Claims 1-12, further comprising a co-initiator; preferably wherein the photoinitiator is riboflavin and the co-initiator is L-arginine.

14. The ocular composition of any one of Claims 1-13, wherein the ocular composition is a nanoparticle or a microparticle ocular implant; preferably wherein the nanoparticle ocular implant is less than 1,000 nm; preferably wherein the microparticle ocular implant is less than 1,000 µm.

15. A method of making the ocular composition of any of the preceding claims comprising the steps of:
i) mixing the therapeutic agent, the photopolymerizable composition, the biodegradable polymer and the photoinitiator, in any order of addition, to form mixture i);
ii) irradiating the mixture i) with light at a wavelength of between 230 to 550 nm, between 300 to 525 nm, or between 350 to 490 nm for between 1 second and 60 minutes to form the ocular composition; and
iii) wherein the composition ii) is for administration to an ocular area of a subject; or
the method comprising the steps of:
i) mixing the therapeutic agent, the photopolymerizable composition, the biodegradable polymer and the photoinitiator, in any order of addition, to form mixture i);
ii) adding the mixture i) to an aqueous medium to form mixture ii);
iii) sonicating the mixture ii); and
iv) irradiating the mixture ii) with light at a wavelength of between 230 to 550 nm, between 300 to 525 nm, or between 350 to 490 nm for between 1 second and 60 minutes to form nanoparticles or microparticles.

16. The ocular composition of Claim 1 wherein the photopolymerizable composition has a molecular weight in the range of 200 to 1,000 Dalton.

17. An ocular composition of any of claims 1-3, or 5-13 or 16 obtainable by a method comprising the step of mixing the therapeutic agent, the photopolymerizable composition, the biodegradable polymer and the photoinitiator, in any order of addition, to form a mixture for use in the ocular area of a subject, wherein, after administration, the mixture is irradiated with light at a wavelength of between 230 to 550 nm, between 300 to 525 nm, or between 350 to 490 nm for between 1 second and 60 minutes to form the ocular composition.

## Patentansprüche

1. Injizierbare Zusammensetzung für das Auge, umfassend:
i) zu 99 bis 60 Gew.-% eine photopolymerisierbare Zusammensetzung, ausgewählt aus der Gruppe bestehend aus Polyalkylenglycoldiacrylat und Polyalkylenglycoldimethacrylat, wobei die photopolymerisierbare Zusammensetzung ein Molekulargewicht im Bereich von 200 bis 20.000 Dalton aufweist;
ii) ein biologisch abbaubares Polymer, ausgewählt aus der Gruppe bestehend aus Lactid/Glycolid-Copolymer (PLGA), Polymilchsäure (PLA), Polyglycolsäure (PGA), Polycaprolacton (PCL), Lactid/Caprolacton-Copolymer (PLC), Poly-(L-lactid) (PLLA) und Gemische, Copolymere und Blockcopolymere davon;
iii) einen Photoinitiator; und
iv) ein therapeutisches Mittel.

2. Zusammensetzung für das Auge nach Anspruch 1 zur Verwendung in einem Augenimplantat oder zur Verwendung zum Beschichten eines Augenimplantats, wobei das Implantat wahlweise ein an Ort und Stelle gebildetes Augenimplantat ist.

3. Zusammensetzung für das Auge nach Anspruch 2, wobei das Implantat ein an Ort und Stelle gebildetes Augenimplantat ist, wobei die photopolymerisierbare Zusammensetzung ein Molekulargewicht im Bereich von 200 bis 20.000 Dalton aufweist, mehr bevorzugt im Bereich von 200 bis 3.000 Dalton oder 200 bis 1.000 Dalton.

4. Zusammensetzung für das Auge nach Anspruch 2, wobei das Implantat ein vorgeformtes Augenimplantat ist.

5. Zusammensetzung für das Auge nach Anspruch 1, wobei das biologisch abbaubare Polymer PLGA ist oder wobei das biologisch abbaubare Polymer ausgewählt ist aus der Gruppe PCL, PLC, PLLA und Gemische, Copolymere und Blockcopolymere davon.

6. Zusammensetzung für das Auge nach einem der Ansprüche 1 bis 5, wobei die photopolymerisierbare Zusammensetzung Polyalkylenglycoldiacrylat ist oder wobei die photopolymerisierbare Zusammensetzung ausgewählt ist aus der Gruppe bestehend aus Polyethylenglycoldiacrylat, Diethylenglycoldiacrylat, Polyethylenglycoldimethacrylat, Diethylenglycoldimethacrylat, Polypropylenglycoldiacrylat, Dipropylenglycoldiacrylat, Dipropylenglycoldimethacrylat und Polypropylenglycoldimethacrylat; oder wobei die photopolymerisierbare Zusammensetzung Polyethylenglycoldiacrylat oder Polyethylenglycoldimethacrylat ist; oder wobei die photopolymerisierbare Zusammensetzung Polyethylenglycoldiacrylat ist.

7. Zusammensetzung für das Auge nach Anspruch 5, wobei das Molverhältnis von Milchsäure zu Glycolsäure in der PLGA 90 % Milchsäure zu 10 % Glycolsäure, 85 % Milchsäure zu 15 % Glycolsäure, 75 % Milchsäure zu 25 % Glycolsäure, 65 % Milchsäure zu 35 % Glycolsäure, 50 % Milchsäure zu 50 % Glycolsäure, 35 % Milchsäure zu 65 % Glycolsäure, 25 % Milchsäure zu 75 % Glycolsäure, 15 % Milchsäure zu 85 % Glycolsäure und 10 % Milchsäure zu 90 % Glycolsäure beträgt.

8. Zusammensetzung für das Auge nach Anspruch 5, umfassend:
i) zu 79,5 bis 59,5 Gew.-% Polyethylenglycoldiacrylat oder Polyethylenenglycoldimethacrylat; und
ii) zu 20 bis 40 Gew.-% PLGA, wobei das Molverhältnis von Milchsäure zu Glycolsäure in der PLGA 90 % Milchsäure zu 10 % Glycolsäure, 85 % Milchsäure zu 15 % Glycolsäure, 75 % Milchsäure zu 25 % Glycolsäure oder 50 % Milchsäure zu 50 % Glycolsäure beträgt; oder
i) zu 69,5 Gew.-% Polyethylenglycoldiacrylat oder Polyethylenglycoldimethacrylat; und
ii) zu 30 Gew.-% PLGA, wobei das Molverhältnis von Milchsäure zu Glycolsäure in der PLGA 90 % Milchsäure zu 10 % Glycolsäure, 85 % Milchsäure zu 15 % Glycolsäure, 75 % Milchsäure zu 25 % Glycolsäure oder 50 % Milchsäure zu 50 % Glycolsäure beträgt; oder
i) zu 95,5 bis 84,5 Gew.-% Polyalkylenglycoldiacrylat oder Polyalkylenglycoldimethacrylat; und
ii) zu 4 bis 15 Gew.-% PCL; oder
i) zu 79,5 bis 94,5 Gew.-% Polyalkylenglycoldiacrylat oder Polyalkylenglycoldimethacrylat; und
ii) zu 5 bis 20 Gew.-% PLLA; oder
i) zu 95,5 bis 84,5 Gew.-% Polyalkylenglycoldiacrylat oder Polyalkylenglycoldimethacrylat; und
ii) zu 4 bis 15 Gew.-% PLC, wobei Milchsäure zu Caprolacton im Bereich von 90 % Milchsäure zu 10 % Caprolacton, 80 % Milchsäure zu 20 % Caprolacton, 70 % Milchsäure zu 30 % Caprolacton, 60 % Milchsäure zu 40 % Caprolacton oder 50 % Milchsäure zu 50 % Caprolacton liegt.

9. Zusammensetzung für das Auge nach einem der Ansprüche 1 bis 8, ferner umfassend ein Lösungsmittel ausgewählt aus Dimethylsulfoxid, Decylmethylsulfoxid, 2-Pyrrolidon, 1-Methyl-2-pyrrolidon, N-Vinyl-pyrrolidin, N-Methyl-2-pyrrolidon, N-Ethyl-pyrrolidon, Glycerolformal, Glycerol, Polyethylenglycol, Propylenglycol, Benzylalkohol, Benzylbenzoat, Ethylbenzoat, Triacetin, Triethylcitrat, Dimethylformamid, Dimethylacetamid und Tetrahydrofuran; wobei das Lösungsmittel vorzugsweise ausgewählt ist aus Dimethylsulfoxid, Decylmethylsulfoxid, 2-Pyrrolidon, N-Methyl-2-pyrrolidon und Glycerolformal.

10. Zusammensetzung für das Auge nach einem der Ansprüche 1 bis 9, ferner umfassend einen Porenbildner;
wobei der Porenbildner vorzugsweise ausgewählt ist aus Polyethylenglycol, Maltose, Glucose, Agarose, Mannitol, Gelatine, Natriumchlorid, Magnesiumcarbonat, Magnesiumhydroxid, Kaliumchlorid, Natriumbicarbonat, Kaliumbicarbonat und Saccharose.

11. Zusammensetzung für das Auge nach einem der Ansprüche 1 bis 10, wobei das biologisch abbaubare Polymer im Wesentlichen in einer Matrix der photopolymerisierbaren Zusammensetzung enthalten ist.

12. Zusammensetzung für das Auge nach einem der Ansprüche 1 bis 11, wobei der Photoinitiator ein Hydroxyketonphotoinitiator, ein Aminoketonphotoinitiator, ein Hydroxyketon-/Benzophenonphotoinitiator, ein Benzyldimethylketalphotoinitiator, ein Acylphosphinoxidphotoinitiator, ein Acylphosphineoxid-/alpha-Hydroxyketonphotoinitiator, ein Benzophenonphotoinitiator, ein Ribitylisoalloxazinphotoinitiator oder ein Phenylglyoxylatphotoinitiator oder eine beliebige Kombination davon ist; wobei der Photoinitiator mehr bevorzugt 1-[4-(2-Hydroxyethoxy)-phenyl]-2-hydroxy-2-methyl-1-propanon, 2,2-Dimethoxy-2-phenylacetophenon (DMPA) oder Riboflavin ist.

13. Zusammensetzung für das Auge nach einem der Ansprüche 1 bis 12, ferner umfassend einen Coinitiator; wobei der Photoinitiator vorzugsweise Riboflavin und der Coinitiator L-Arginin ist.

14. Zusammensetzung für das Auge nach einem der Ansprüche 1 bis 13, wobei die Zusammensetzung für das Auge ein Nanopartikel- oder ein Mikropartikelaugenimplantat ist; wobei vorzugsweise das Nanopartikelaugenimlantat kleiner als 1.000 nm ist; wobei vorzugsweise das Mikropartikelaugenimplantat kleiner als 1.000 µm ist.

15. Verfahren zum Herstellen der Zusammensetzung für das Auge nach einem der vorangehenden Ansprüche, folgende Schritte umfassend:
i) Mischen des therapeutischen Mittels, der photopolymerisierbaren Zusammensetzung, des biologisch abbaubaren Polymers und des Photoinitiators in einer beliebigen Zusetzungsreihenfolge, um ein Gemisch i) zu bilden;
ii) Bestrahlen des Gemischs i) mit Licht bei einer Wellenlänge von zwischen 230 und 550 nm, zwischen 300 und 525 nm oder zwischen 350 und 490 nm für zwischen 1 Sekunde und 60 Minuten, um die Zusammensetzung für das Auge zu bilden; und
iii) wobei die Zusammensetzung ii) zur Verabreichung an einen Augenbereich eines Individuums vorgesehen ist; oder das Verfahren folgende Schritte umfasst:
i) Mischen des therapeutischen Mittels, der photopolymerisierbaren Zusammensetzung, des biologisch abbaubaren Polymers und des Photoinitiators in einer beliebigen Zusetzungsreihenfolge, um ein Gemisch i) zu bilden;
ii) Zusetzen des Gemischs i) zu einem wässrigen Medium, um Gemisch ii) zu bilden;
iii) Behandeln des Gemischs ii) im Ultraschallbad; und
iv) Bestrahlen des Gemischs i) mit Licht bei einer Wellenlänge von zwischen 230 und 550 nm, zwischen 300 und 525 nm oder zwischen 350 und 490 nm für zwischen 1 Sekunde und 60 Minuten, um Nanopartikel oder Mikropartikel zu bilden.

16. Zusammensetzung für das Auge nach Anspruch 1, wobei die photopolymerisierbare Zusammensetzung ein Molekulargewicht im Bereich von 200 bis 1.000 Dalton aufweist.

17. Zusammensetzung für das Auge nach einem der Ansprüche 1 bis 3 oder 5 bis 13 oder 16, die durch ein Verfahren erlangbar ist, das den Schritt Mischen des therapeutischen Mittels, der photopolymerisierbaren Zusammensetzung, des biologisch abbaubaren Polymers und des Photoinitiators in einer beliebigen Zusetzungsreihenfolge zum Bilden eines Gemischs zur Verwendung im Augenbereich eines Individuums umfasst, wobei das Gemisch nach der Verabreichung mit Licht bei einer Wellenlänge von zwischen 230 und 550 nm, zwischen 300 und 525 nm oder zwischen 350 und 490 nm für zwischen 1 Sekunde und 60 Minuten bestrahlt wird, um die Zusammensetzung für das Auge zu bilden.

## Revendications

1. Composition oculaire injectable comprenant :
i) 99 à 60 % (p/p) d'une composition photopolymérisable choisie dans le groupe constitué du diacrylate de polyalkylène glycol et du diméthacrylate de polyalkylène glycol, dans laquelle la composition photopolymérisable a un poids moléculaire dans la plage de 200 à 20 000 daltons ;
ii) un polymère biodégradable choisi dans le groupe constitué du copolymère lactide/glycolide (PLGA), de l'acide polylactique (PLA), de l'acide polyglycolique (PGA), de la polycaprolactone (PCL), du copolymère lactide/caprolactone (PLC), du poly (L-lactide) (PLLA) et de leurs mélanges, copolymères et copolymères séquencés ;
iii)un photoinitiateur ; et
iv) un agent thérapeutique.

2. Composition oculaire selon la revendication 1, à utiliser pour former un implant oculaire ou à utiliser pour revêtir un implant oculaire, éventuellement dans laquelle l'implant est un implant oculaire formé *in situ.*

3. Composition oculaire selon la revendication 2, dans laquelle l'implant est un implant oculaire formé *in situ,* dans laquelle la composition photopolymérisable a un poids moléculaire dans la plage de 200 à 20 000 daltons, plus préférablement dans la plage de 200 à 3 000 daltons ou de 200 à 1 000 daltons.

4. Composition oculaire selon la revendication 2, dans laquelle l'implant est un implant oculaire préformé.

5. Composition oculaire selon la revendication 1, dans laquelle le polymère biodégradable est le PLGA ou dans laquelle le polymère biodégradable est choisi dans le groupe PCL, PLC, PLLA et leurs mélanges, copolymères et copolymères séquences.

6. Composition oculaire selon l'une quelconque des revendications 1 à 5, dans laquelle la composition photopolymérisable est le diacrylate de polyalkylène glycol ou dans laquelle la composition photopolymérisable est choisie dans le groupe constitué du diacrylate de polyéthylène glycol, du diacrylate de diéthylène glycol, du diméthacrylate de polyéthylène glycol, du diméthacrylate de diéthylène glycol, du diacrylate de polypropylène glycol, du diacrylate de dipropylène glycol, du diméthacrylate de dipropylène glycol et du diméthacrylate de polypropylène glycol ; ou, dans laquelle la composition photopolymérisable est le diacrylate de polyéthylène glycol ou le diméthacrylate de polyéthylène glycol ; ou dans laquelle la composition photopolymérisable est le diacrylate de polyéthylène glycol.

7. Composition oculaire selon la revendication 5, dans laquelle le rapport molaire de l'acide lactique à l'acide glycolique dans le PLGA est de 90 % d'acide lactique à 10 % d'acide glycolique, 85 % d'acide lactique à 15 % d'acide glycolique, 75 % d'acide lactique à 25 % d'acide glycolique, 65 % d'acide lactique à 35 % d'acide glycolique, 50 % d'acide lactique à 50 % d'acide glycolique, 35 % d'acide lactique à 65 % d'acide glycolique, 25 % d'acide lactique à 75 % d'acide glycolique, 15 % d'acide lactique à 85 % d'acide glycolique et 10 % d'acide lactique à 90 % d'acide glycolique.

8. Composition oculaire selon la revendication 5, comprenant :
i) 79,5 à 59,5 % (p/p) de diacrylate de polyéthylène glycol ou de diméthacrylate de polyéthylène glycol ; et
ii) 20 à 40 % (p/p) de PLGA, dans laquelle le rapport molaire de l'acide lactique à l'acide glycolique dans le PLGA est de 90 % d'acide lactique à 10 % d'acide glycolique, 85 % d'acide lactique à 15 % d'acide glycolique, 75 % d'acide lactique à 25 % d'acide glycolique ou 50 % d'acide lactique à 50 % d'acide glycolique ; ou
i) 69,5 % (p/p) de diacrylate de polyéthylène glycol ou de diméthacrylate de polyéthylène glycol ; et
ii) 30 % (p/p) de PLGA dans laquelle le rapport molaire de l'acide lactique à l'acide glycolique dans le PLGA est de 90 % d'acide lactique à 10 % d'acide glycolique, 85 % d'acide lactique à 15 % d'acide glycolique, 75 % d'acide lactique à 25 % d'acide glycolique ou 50 % d'acide lactique à 50 % d'acide glycolique ; ou
i) 95,5 à 84,5 % (p/p) de diacrylate de polyalkylène glycol ou de diméthacrylate de polyalkylène glycol ; et
ii) 4 à 15 % (p/p) de PCL ; ou
i) 79,5 à 94,5 % (p/p) de diacrylate de polyalkylène glycol ou de diméthacrylate de polyalkylène glycol ; et
ii) 5 à 20 % (p/p) de PLLA ; ou
i) 95,5 à 84,5 % (p/p) de diacrylate de polyalkylène glycol ou de diméthacrylate de polyalkylène glycol ; et
ii) 4 à 15 % (p/p) de PLC dans laquelle l'acide lactique par rapport à la caprolactone est dans la plage de 90 % d'acide lactique à 10 % de caprolactone, 80 % d'acide lactique à 20 % de caprolactone, 70 % d'acide lactique à 30 % de caprolactone, 60 % d'acide lactique à 40 % de caprolactone ou 50 % d'acide lactique à 50 % de caprolactone.

9. Composition oculaire selon l'une quelconque des revendications 1 à 8, comprenant en outre un solvant choisi parmi le diméthylsulfoxyde, le décylméthylsulfoxyde, la 2-pyrrolidone, la 1-méthyl-2-pyrrolidone, la N-vinyl-pyrrolidine, la N-méthyl-2-pyrrolidone, la N-éthyl-pyrrolidone, le glycérol formai, le glycérol, le polyéthylène glycol, le propylène glycol, l'alcool benzylique, le benzoate de benzyle, le benzoate d'éthyle, la triacétine, le citrate de triéthyle, le diméthylformamide, le diméthylacétamide et le tétrahydrofuranne ; de préférence dans laquelle le solvant est choisi parmi le diméthylsulfoxyde, le décylméthylsulfoxyde, la 2-pyrrolidone, la 1-méthyl-2-pyrrolidone, la N-méthyl-2-pyrrolidone et le glycérol formai.

10. Composition oculaire selon l'une quelconque des revendications 1 à 9, comprenant en outre un agent porogène ; de préférence dans laquelle l'agent porogène est choisi parmi le polyéthylène glycol, le maltose, le glucose, l'agarose, le mannitol, la gélatine, le chlorure de sodium, le carbonate de magnésium, l'hydroxyde de magnésium, le chlorure de potassium, le bicarbonate de sodium, le bicarbonate de potassium et le saccharose.

11. Composition oculaire selon l'une quelconque des revendications 1 à 10, dans laquelle le polymère biodégradable est essentiellement contenu à l'intérieur d'une matrice de la composition photopolymérisable.

12. Composition oculaire selon l'une quelconque des revendications 1 à 11, dans laquelle le photoinitiateur est un photoinitiateur d'hydroxycétone, un photoinitiateur d'aminocétone, un photoinitiateur d'hydroxycétone/benzophénone, un photoinitiateur de benzyldiméthylcétal, un photoinitiateur d'oxyde d'acylphosphine, un photoinitiateur d'oxyde d'acylphosphine/alpha hydroxy cétone, un photoinitiateur de benzophénone, un photoinitiateur de ribityle isoalloxazine ou un photoinitiateur de phénylglyoxylate ou toute combinaison de ceux-ci ; plus préférablement dans laquelle le photoinitiateur est la 1-[4-(2-hydroxyéthoxy)-phényl]-2-hydroxy-2-méthyl-l-propanone, la 2,2-diméthoxy-2-phénylacétophénone (DMPA) ou la riboflavine.

13. Composition oculaire selon l'une quelconque des revendications 1 à 12, comprenant en outre un co-initiateur ; de préférence dans laquelle le photoinitiateur est la riboflavine et le co-initiateur est la L-arginine.

14. Composition oculaire selon l'une quelconque des revendications 1 à 13, dans laquelle la composition oculaire est un implant oculaire à nanoparticules ou à microparticules ; de préférence dans laquelle l'implant oculaire à nanoparticules est inférieur à 1 000 nm ; de préférence dans laquelle l'implant oculaire à microparticules est inférieur à 1 000 µm.

15. Procédé de fabrication de la composition oculaire selon l'une quelconque des revendications précédentes comprenant les étapes :
i) de mélange de l'agent thérapeutique, de la composition photopolymérisable, du polymère biodégradable et du photoinitiateur, dans n'importe quel ordre d'addition, pour former le mélange i) ;
ii) d'irradiation du mélange i) avec de la lumière à une longueur d'onde comprise entre 230 et 550 nm, entre 300 et 525 nm ou entre 350 et 490 nm pendant entre 1 seconde et 60 minutes pour former la composition oculaire ; et
iii) dans laquelle la composition ii) est destinée à être administrée à une zone oculaire d'un sujet ; ou
le procédé comprenant les étapes :
i) de mélange de l'agent thérapeutique, de la composition photopolymérisable, du polymère biodégradable et du photoinitiateur, dans n'importe quel ordre d'addition, pour former le mélange i) ;
ii) d'ajout du mélange i) à un milieu aqueux pour former le mélange ii) ;
iii) de sonication du mélange ii) ; et
iv) d'irradiation du mélange ii) avec de la lumière à une longueur d'onde comprise entre 230 à 550 nm, entre 300 à 525 nm ou entre 350 à 490 nm pendant entre 1 seconde et 60 minutes pour former des nanoparticules ou des microparticules.

16. Composition oculaire selon la revendication 1, dans laquelle la composition photopolymérisable a un poids moléculaire dans la plage de 200 à 1 000 daltons.

17. Composition oculaire selon l'une quelconque des revendications 1 à 3, ou 5 à 13 ou 16 pouvant être obtenue par un procédé comprenant l'étape de mélange de l'agent thérapeutique, de la composition photopolymérisable, du polymère biodégradable et du photoinitiateur, dans n'importe quel ordre d'addition, pour former un mélange destiné à être utilisé dans la zone oculaire d'un sujet, dans laquelle, après administration, le mélange est irradié avec de la lumière à une longueur d'onde comprise entre 230 et 550 nm, entre 300 et 525 nm ou entre 350 et 490 nm pendant entre 1 seconde et 60 minutes pour former la composition oculaire.
